(19) Europäisches Patentamt
European Patent Office
Office européen des brevets

(11) Veröffentlichungsnummer: **0 107 620**
**A1**

(12) # EUROPÄISCHE PATENTANMELDUNG

(21) Anmeldenummer: **83810462.8**

(22) Anmeldetag: **07.10.83**

(51) Int. Cl.[3]: **C 07 C 91/44,** C 07 C 93/26, C 07 C 95/08, C 07 D 207/325, C 07 D 295/08, C 07 D 295/10, A 61 K 31/135, A 61 K 31/22, A 61 K 31/395

(30) Priorität: **13.10.82 CH 5987/82**

(43) Veröffentlichungstag der Anmeldung: **02.05.84 Patentblatt 84/18**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Wenk, Paul, Dr., Quellenweg 7, CH-4123 Allschwil (CH)**
Erfinder: **Breitenstein, Werner, Dr., St. Galler-Ring 179, CH-4054 Basel (CH)**
Erfinder: **Baumann, Marcus, Dr., Hammerstrasse 82, CH-4057 Basel (CH)**

(54) **Phenol-Derivate.**

(57) Die Erfindung betrifft neue Phenol-Derivate, insbesondere solche der allgemeinen Formel

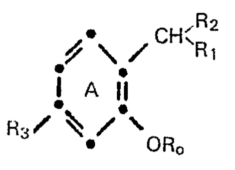

(I),

worin $R_0$ Wasserstoff oder einen Acylrest darstellt, $R_1$ Hydroxymethyl, verestertes oder verethertes Hydroxymethyl, Formyl oder acetalisiertes Formyl bedeutet, $R_2$ für Wasserstoff oder einen aliphatischen Rest steht, $R_3$ eine durch zwei einwertige Kohlenwasserstoffreste oder durch einen zweiwertigen, gegebenenfalls durch mindestens ein Heteroatom unterbrochenen Kohlenwasserstoffrest disubstituierte Aminogruppe darstellt und der aromatische Ring A zusätzlich substituiert sein kann, und ihre Salze und Isomeren, Verfahren zur Herstellung von Verbindungen der Formel (I) und ihre Salze und Isomeren, solche Verbindungen enthaltende pharmazeutische Präparate sowie deren Verwendung als Arzneimittelwirkstoffe und/oder zur Herstellung pharmazeutischer Präparate.

EP 0 107 620 A1

CIBA-GEIGY AG
Basel (Schweiz)

4-14146/ZFO/+

## Phenol-Derivate

Die Erfindung betrifft neue Phenol-Derivate, insbesondere solche der allgemeinen Formel

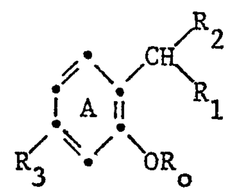

(I),

worin $R_o$ Wasserstoff oder einen Acylrest darstellt, $R_1$ Hydroxymethyl, verestertes oder verethertes Hydroxymethyl, Formyl oder acetalisiertes Formyl bedeutet, $R_2$ für Wasserstoff oder einen aliphatischen Rest steht, $R_3$ eine durch zwei einwertige Kohlenwasserstoffreste oder durch einen zweiwertigen, gegebenenfalls durch mindestens ein Heteroatom unterbrochenen Kohlenwasserstoffrest disubstituierte Aminogruppe darstellt und der aromatische Ring A zusätzlich substituiert sein kann, und ihre Salze und Isomeren, Verfahren zur Herstellung von Verbindungen der Formel (I) und ihrer Salze und Isomeren, solche Verbindungen enthaltende pharmazeutische Präparate sowie deren Verwendung als Arzneimittelwirkstoffe und/oder zur Herstellung pharmazeutischer Präparate.

Ein Acylrest ist beispielsweise ein Niederalkanoylrest oder ein Arylniederalkanoylrest, wie ein im Phenylteil ein- oder mehrfach substituierter bzw. unsubstituierter Phenylniederalkanoylrest, wobei Phenyl z.B. durch einen aliphatischen Rest, wie Niederalkyl, Niederalkenyl, gegebenenfalls verzweigtes, insbesondere zwei C-Atome überbrückendes,

3- bis 4-gliedriges Alkylen, wie Niederalkylen, mit 3 bis 8 C-Atomen, Hydroxyniederalkyl oder Halogenniederalkyl, durch Niederalkoxy, Niederalkylthio, Niederalkansulfinyl, Niederalkansulfonyl, Hydroxy, Niederalkanoyloxy, Niederalkanoyl, Halogen und/oder Nitro ein- oder mehrfach substituiert oder unsubstituiert sein kann.

Mit einer Carbonsäure verestertes Hydroxymethyl ist z.B. mit einer gegebenenfalls substituierten Niederalkancarbonsäure, z.B. Essigsäure, verestertes Hydroxymethyl, während mit einem Alkohol verethertes Hydroxymethyl z.B. mit einem Niederalkanol, wie Methanol oder Ethanol, verethertes Hydroxymethyl darstellt.

Acetalisiertes Formyl bedeutet z.B. mit Niederalkanolen oder einem Niederalkandiol acetalisiertes Formyl, wie Diniederalkoxy-, z.B. Dimethoxy- oder Diethoxy-methyl, oder Niederalkylen-, z.B. Ethylen- oder Trimethylendioxy-methyl.

Ein aliphatischer Rest $R_2$ ist insbesondere gesättigt und unsubstituiert und stellt in erster Linie einen Niederalkylrest dar.

Der aromatische Ring A kann zusätzlich, z.B. durch einen aliphatischen Rest, wie Niederalkyl, Hydroxyniederalkyl, Halogenniederalkyl, Niederalkenyl oder gegebenenfalls verzweigtes, insbesondere zwei benachbarte C-Atome überbrückendes, 3- bis 4-gliedriges Alkylen, wie Niederalkylen, durch Niederalkoxy, Hydroxy, Niederalkylthio, Niederalkansulfinyl, Niederalkansulfonyl, Halogen, Niederalkanoyloxy, Niederalkanoyl und/oder Nitro, ein- oder mehrfach substituiert oder, bis auf $R_3$, unsubstituiert sein.

BAD ORIGINAL

Eine durch zwei einwertige Kohlenwasserstoffreste disubstituierte Aminogruppe weist als solche z.B. einwertige aliphatische Reste, wie Niederalkylreste auf, die unsubstituiert oder durch 3- bis 7-gliedriges Cycloalkyl oder durch Aryl, wie unsubstituiertes bzw. z.B. einen aliphatischen Rest, wie Niederalkyl, Niederalkenyl, Niederalkylen, Hydroxyniederalkyl, Halogenniederalkyl, sowie Niederalkoxy, Niederalkylthio, Niederalkansulfinyl, Niederalkansulfonyl, Hydroxy, Halogen, Niederalkanoyloxy, Niederalkanoyl und/oder Nitro aufweisendes Phenyl, substituiert sein können. Eine durch einen zweiwertigen Kohlenwasserstoffrest disubstituierte Aminogruppe weist als solchen z.B. einen zweiwertigen aliphatischen Rest auf, der auch durch Aza, N-Niederalkylaza, Oxa oder Thia unterbrochen sein kann, wie Niederalkylen, Niederalkenylen, z.B. mit einer oder zwei Doppelbindungen, jeweils durch Aza, N-Niederalkylaza, Oxa, Thia unterbrochenes Niederalkylen bzw. Niederalkenylen, wobei Niederalkylen bzw. Niederalkenylen jeweils auch verzweigt sein können und 4 bis und mit 14, insbesondere 4 bis und mit 7, C-Atomen aufweisen. Ferner können derartige cyclische Amine $R_3$ auch ein oder zwei ortho-anellierte Benzosysteme aufweisen.

$R_3$ bedeutet vorzugsweise Di-niederalkyl-amino, Cycloalkylniederalkylniederalkyl-amino, Di-cycloalkylniederalkyl-amino, Niederalkyl-phenylniederalkyl-amino, Di-phenylniederalkyl-amino, ferner Phenylniederalkyl-cycloniederalkyl-amino, sowie jeweils 5- bis 8-gliedriges, insbesondere 5- oder 6-gliedriges, Niederalkylenamino, Niederalkenylenamino, z.B. mit einer oder zwei Doppelbindungen, Azaniederalkylenamino, N'-Niederalkylaza-niederalkylenamino, Oxaniederalkylenamino, Thianiederalkylenamino, ferner Azaniederalkenylenamino, N'-Niederalkylazaniederalkenylenamino, Oxaniederalkenylenamino oder Thianiederalkenylenamino, wobei Niederalkylen- bzw. Niederalkenylenamino auch verzweigt sein und entsprechend 4 bis 14, vorzugsweise 4 bis 7 C-Atome aufweisen kann. Als Beispiele für derartige Reste $R_3$ seien genannt:

Pyrrolidin-1-yl, 2- oder 3-Pyrrolin-1-yl Pyrrol-1-yl, Piperidin-1-yl, Perhydroazepin-1-yl, Piperazin-1-yl, Morpholin-4-yl, Thiomorpholin-4-yl, ferner 3-Methyl-imidazolin-1-yl, 4-Methylpiperazin-1-yl, Imidazolidin-1-yl, 2-, 3- oder 4-Imidazolin-1-yl, Oxazolidin-3-yl, 4-Oxazolin-3-yl, Thiazolidin-3-yl, 4-Thiazolin-3-yl,.

Ferner bedeutet $R_3$ Niederalkylen- bzw. Niederalkenylen-amino, welches ein oder zwei ortho-anellierte Benzosysteme aufweist, wie Indol-1-yl, Indolin-1-yl, Isoindol-2-yl, Isoindolin-2-yl, Carbazol-9-yl oder β-Carbolin-9-yl.

Vor- und nachstehend sind unter mit "nieder" bezeichneten organischen Resten und Verbindungen vorzugsweise solche zu verstehen, die bis und mit 7, vor allem bis und mit 4 Kohlenstoffatome enthalten.

Die im Rahmen des vorliegenden Textes verwendeten Allgemeindefinitionen haben in erster Linie die folgenden Bedeutungen:

Niederalkyl ist z.B. Methyl, Ethyl, n-Propyl, Isopropyl, n-Butyl, Isobutyl, sek.-Butyl, tert.-Butyl und umfasst ferner entsprechende Pentyl-, Hexyl- oder Heptylreste.

Hydroxyniederalkyl ist z.B. Hydroxymethyl, 2-Hydroxyethyl oder 2- oder 3-Hydroxypropyl.

Halogenniederalkyl ist z.B. Chlormethyl oder Trifluormethyl.

Niederalkenyl ist z.B. 2-Propenyl, 2- oder 3-Butenyl oder 1,3-Butadienyl.

3- oder 4-gliedriges Niederalkylen ist z.B. geradkettig, wie Tri- oder Tetramethylen, oder verzweigt, wie 2,4-Butylen, 2,4-Pentylen oder 2-Methyl-1,3-propylen.

Niederalkoxy ist z.B. Methoxy, Ethoxy, n-Propyloxy, Isopropyloxy, n-Butyloxy, Isobutyloxy, sek.-Butyloxy, tert.-Butyloxy, ferner entsprechende Pentyloxy-, Hexyloxy- oder Heptyloxyreste.

Niederalkylthio ist z.B. Methyl-, Ethyl-, n-Propyl-, Isopropyl-, n-Butyl-, Isobutyl-, sek.-Butyl- oder tert.-Butylthio.

Niederalkansulfinyl bzw. -sulfonyl ist z.B. Methan-, Ethan-, n-Propan- oder Isopropan-sulfinyl bzw. -sulfonyl.

Halogen ist z.B. Halogen der Atomnummer bis und mit 35, wie Fluor, Chlor oder Brom, und umfasst ferner Iod.

Niederalkanoyloxy ist z.B. Acetyloxy, Propionyloxy, Butyryloxy, Isobutyryloxy, sek.-Butyryloxy oder tert.-Butyryloxy.

Niederalkanoyl ist z.B. Acetyl, Propionyl, Butyryl, Isobutyryl oder tert.-Butyryl.

3- bis 7-gliedriges Cycloalkyl ist z.B. Cyclopropyl, Cyclobutyl, Cyclopentyl, Cyclohexyl oder Cycloheptyl.

Niederalkylen weist z.B. 4 bis und mit 10, insbesondere 4 oder 5, C-Atome auf und ist z.B. geradkettig, wie 4- bis 7-gliedriges Niederalkylen, wie Tetra-, Penta- oder Hexamethylen, ferner Heptamethylen, oder verzweigt, wie 2,3-Dimethyl- oder 2,3-Diethyl-1,4-butylen.

Durch Aza bzw. N-Niederalkylaza unterbrochenes Niederalkylen ist z.B. 4- bis 7-gliedriges Monoaza- bzw. N'-Niederalkylmonoaza-niederalkylen, wie 2-Aza-tetramethylen, 3-Aza-pentamethylen oder 3-Methylazapentamethylen.

Durch Oxa bzw. Thia unterbrochenes Niederalkylen ist z.B. Monooxa- bzw. Monothia-niederalkylen, wie 3-Oxa- bzw. 3-Thia-pentamethylen.

Niederalkenylen weist z.B. 4 bis und mit 10, insbesondere 4 oder 5, C-Atome und eine oder mehrere, z.B. zwei, Doppelbindungen auf und ist z.B. 4- bis 7-gliedriges Niederalkenylen, wie But-2-en-1,4-ylen, Buta-1,3-dien-1,4-ylen, Pent-2-en-1,5-ylen oder Penta-1,3-dien-1,5-ylen, Penta-1,4-dien-1,5-ylen, Hex-3-en-2,5-ylen oder Hexa-2,4-dien-2,4-ylen.

Durch Aza bzw. N-Niederalkylaza unterbrochenes Niederalkenylen mit einer oder mehreren Doppelbindungen ist z.B. 2-Aza-buten-1-ylen, 2-Aza-buten-2-ylen, 2-Aza-buten-3-ylen, 2-Methylaza-buten-3-ylen oder 2-Aza-butadien-1,3-ylen.

Die erfindungsgemässen Verbindungen der Formel (I) können, falls $R_3$ genügend basisch ist, vorzugsweise pharmazeutisch verwendbare Salze, wie pharmazeutisch verwendbare Säureadditionssalze, bilden. Diese werden beispielsweise mit starken anorganischen Säuren, wie Mineralsäuren, z.B. Schwefelsäure, Phosphorsäure oder Halogenwasserstoffsäuren, mit starken organischen Carbonsäuren, wie Niederalkancarbonsäuren, z.B. Eisessig, wie gegebenenfalls ungesättigte Dicarbonsäuren, z.B. Oxal-, Malon-, Malein- oder Fumarsäure, oder wie Hydroxycarbonsäuren, z.B. Weinsäure oder Citronensäure, oder mit Sulfonsäuren, wie Niederalkan- oder gegebenenfalls substituierte Benzolsulfonsäure, z.B. Methan- oder p-Toluolsulfonsäure,gebildet. Umfasst sind ferner für pharmazeutische Verwendungen ungeeignete Salze, da letztere beispielsweise für die Isolierung bzw. Reinigung freier erfindungsgemässer Verbindungen sowie oder zur Herstellung deren pharmazeutisch verwendbaren Salze verwendet werden können.

BAD ORIGINAL

Isomere von Verbindungen der Formel I, worin $R_1$ für Formyl steht und $R_o$ Wasserstoff ist, sind beispielsweise Verbindungen der Formel

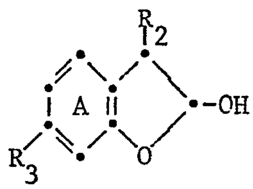

(I') ,

die im Gleichgewicht mit den entsprechenden offenen Formen der Formel I stehen und ebenfalls einen Gegenstand der Erfindung bilden.

Die Verbindungen der Formel (I) weisen wertvolle pharmakologische Eigenschaften auf. Insbesondere besitzen sie eine ausgeprägte antiinflammatorische Wirkung, die sich z.B. durch Hemmung des durch Carrageenin erzeugten Pfotenoedems bei der Ratte bei einer Dosis von etwa 0,1 bis etwa 100 mg/kg p.o. analog der von Pasquale et al., Ag. and Actions, 5, 256 (1975), beschriebenen Methode sowie im Adjuvans-Arthritis-Modell an der Ratte bei einer Dosis von etwa 0,1 bis etwa 100 mg/kg p.o. analog L.Riesterer et al., Pharmacology, 2, 288 (1969), nachweisen lässt. Ausserdem hemmen Verbindungen der Formel (I) die Prostaglandinsynthese aus Arachidonsäure und weisen somit ebenfalls antipyretische Eigenschaften auf.

Weiterhin weisen die Verbindungen der Formel (I) eine deutliche antinociceptive Wirkungskomponente auf, die sich z.B. aus der von L.C. Hendershot et al., J. Pharmacol. exp. Therap., 125, 237 (1959) beschriebenen Reduktion des durch Phenyl-p-Benzochinon induzierten Writhing-Syndroms an der Maus bei einer Dosis von etwa 1 bis etwa 100 mg/kg p.o. ableiten lässt.

Ferner zeigen die Verbindungen der Formel (I) die Fähigkeit, aus dem Bereich des UV-Spektrums die auf der Epidermis Erythreme erzeugenden Strahlen (zwischen 290 und 320 nm) zu absorbieren, während die Substanzen für die bräunend wirkenden Strahlen von etwa 320 bis 400 nm durchlässig sind.

Infolgedessen lassen sich diese Verbindungen als Antiinflammatorika, und/oder (periphere) Analgetika, ferner als Antipyretika, verwenden. Zudem sind diese Verbindungen zur Verwendung als Lichtschutzmittel, z.B. für kosmetische Zwecke, geeignet.

Die Erfindung betrifft beispielsweise Verbindungen der Formel (I), worin $R_o$ Wasserstoff, einen Niederalkanoyl- oder einen Arylniederalkanoylrest bedeutet, $R_1$ Hydroxymethyl, mit einer Carbonsäure verestertes oder mit einem Alkohol verethertes Hydroxymethyl, Formyl oder mit einem Alkohol acetalisiertes Formyl, darstellt, $R_2$ einen gesättigten und unsubstituierten aliphatischen Rest bedeutet, $R_3$ eine durch zwei einwertige aliphatische Reste disubstituierte Aminogruppe oder eine durch einen zweiwertigen, gegebenenfalls durch Aza, N-Niederalkylaza, Oxa oder Thia unterbrochenen aliphatischen Rest disubstituierte Aminogruppe darstellt und der aromatische Ring A zusätzlich durch einen aliphatischen Rest, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_3$, unsubstituiert sein kann, und ihre Salze, insbesondere pharmazeutisch verwendbare Salze, und Isomeren.

Die Erfindung betrifft beispielsweise Verbindungen der Formel (I), worin $R_o$ Wasserstoff, Niederalkanoyl oder Phenylniederalkanoyl bedeutet, wobei der Phenylrest ein- oder mehrfach durch Niederalkyl, Hydroxyniederalkyl, Halogenniederalkyl, Niederalkenyl, 3- oder 4-gliedriges Niederalkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann,

$R_1$ Hydroxymethyl, mit einer gegebenenfalls substituierten Niederalkancarbonsäure verestertes oder mit einem Niederalkanol verethertes Hydroxymethyl, Formyl oder mit einem Niederalkanol acetalisiertes Formyl darstellt, $R_2$ Wasserstoff oder Niederalkyl bedeutet und $R_3$ einerseits N,N-Di-niederalkyl-amino, N-Cycloniederalkyl-N-niederalkyl-amino, N-Niederalkyl-N-phenylniederalkyl-amino, N,N-Di-cycloniederalkylniederalkyl-amino, N-Cycloniederalkyl-N-phenylniederalkyl-amino oder N,N-Di-phenylniederalkyl-amino bedeutet oder $R_3$ andererseits jeweils 5- bis 8-gliedriges Niederalkylenamino, Niederalkenylenamino, durch Monoaza, N'-Niederalkylmonoaza, Monooxa oder Monothia unterbrochenes Niederalkylenamino, durch Monoaza, N'-Niederalkylmonoaza, Monooxa oder Monothia unterbrochenes Niederalkenylenamino oder ein oder zwei ortho-anellierte Benzosysteme aufweisendes Niederalkylen- bzw. Niederalkenylen-amino darstellt, wobei Niederalkylen bzw. Niederalkenylen unverzweigt oder verzweigt sein und 4 bis 14, insbesondere 4 bis 7, C-Atome aufweisen kann und/oder ein oder zwei ortho-anellierte Benzosysteme aufweisen kann und Phenyl oder Benzo jeweils ein- oder mehrfach durch Niederalkyl, Hydroxyniederalkyl, Halogenniederalkyl, Niederalkenyl, 3- oder 4-gliedriges Niederalkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, und der aromatische Ring A ein- oder mehrfach durch Niederalkyl, Hydroxyniederalkyl, Halogenniederalkyl, Niederalkenyl, 3- oder 4-gliedriges Niederalkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder, bis auf $R_3$, unsubstituiert sein kann, und ihre Salze und Isomeren.

Die Erfindung betrifft insbesondere Verbindungen der Formel

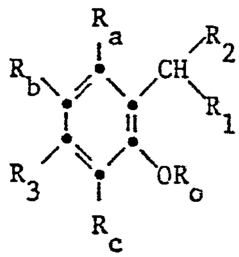

(Ia),

worin $R_o$ Wasserstoff oder Niederalkanoyl, wie Acetyl, bedeutet,
$R_1$ Hydroxymethyl, Niederalkanoyloxymethyl, Niederalkoxymethyl, Formyl oder Diniederalkoxymethyl darstellt,
$R_2$ Wasserstoff oder Niederalkyl, wie Methyl, bedeutet,
$R_3$ einerseits N,N-Di-phenylniederalkyl-amino, wie Dibenzylamino, darstellt oder $R_3$ andererseits jeweils 5- bis 8-gliedriges Niederalkylenamino, wie 1-Piperidino, Niederalkenylenamino, wie 1-Pyrrolyl, durch Monooxa unterbrochenes Niederalkylenamino, wie 4-Morpholino, oder ein ortho-anelliertes Benzosystem aufweisendes Niederalkylenamino bzw. Niederalkenylenamino, wie Indolin-1-yl oder Indol-1-yl bedeutet, und/oder $R_a$, $R_b$ und $R_c$ unabhängig voneinander Wasserstoff, Niederalkyl, wie Methyl, oder Halogen, wie Chlor oder Brom, darstellen, und ihre Salze und Isomeren.

Die Erfindung betrifft vor allem Verbindungen der Formel (Ia), worin $R_o$ Wasserstoff oder Niederalkanoyl, insbesondere mit bis und mit 5 C-Atomen, wie Acetyl, bedeutet, $R_1$ Hydroxymethyl, Niederalkanoyloxymethyl, Niederalkoxymethyl, Formyl oder Diniederalkoxymethyl darstellt, $R_2$ Wasserstoff oder Niederalkyl, insbesondere mit bis und mit 4 C-Atomen, wie Methyl, darstellt, $R_3$ Di-niederalkyl-amino, insbesondere mit bis und mit 4 C-Atomen im Alkylteil, wie N,N-Dimethylamino, 5- bis 8-gliedriges Niederalkylenamino, wie Pyrrolidin-1-yl, 5- bis 8-gliedriges Niederalkenylen-

amino, wie Pyrrol-1-yl, oder 5- bis 8-gliedriges Monooxa-niederalkylenamino, wie Morpholin-4-yl, bedeutet und $R_a$ sowie $R_c$ Wasserstoff bedeuten und $R_b$ Halogen, insbesondere bis und mit Atomnummer 35, wie Chlor, bedeutet und ihre Salze und Isomeren.

Die Erfindung betrifft in erster Linie Verbindungen der Formel (Ia), worin $R_o$ Wasserstoff oder Niederalkanoyl mit bis und mit 5 C-Atomen, wie Acetyl, bedeutet, $R_1$ Hydroxymethyl, Niederalkanoyloxymethyl, Niederalkoxymethyl, Formyl oder Diniederalkoxymethyl darstellt, $R_2$ Niederalkyl mit bis und mit 4 C-Atomen, wie Methyl, bedeutet, $R_3$ 5- bis 7-gliedriges Niederalkylenamino, wie Pyrrolidin-1-yl, Morpholin-4-yl oder Pyrrol-1-yl darstellt, $R_a$ und $R_c$ jeweils Wasserstoff bedeuten und $R_b$ Niederalkyl mit bis und mit 4 C-Atomen, wie Methyl, oder Halogen bis und mit Atomnummer 35, wie Chlor, darstellt, und ihre Salze und Isomeren.

Die Erfindung betrifft in erster Linie Verbindungen der Formel (Ia), worin $R_o$ Niederalkanoyl mit bis und mit 5 C-Atomen, wie Acetyl, darstellt, $R_1$ Hydroxymethyl, Niederalkoxymethyl mit bis und mit 5 C-Atomen oder Formyl, bedeutet, $R_2$ Niederalkyl mit bis und mit 4 C-Atomen, wie Methyl, darstellt, $R_3$ Morpholin-4-yl oder Pyrrol-1-yl ist, $R_a$ und $R_c$ Wasserstoff bedeuten und $R_b$ Halogen bis und mit Atomnummer 35, wie Chlor, oder Niederalkyl mit bis und mit 4 C-Atomen, wie Methyl, darstellt und ihre Salze und Isomeren.

Die Erfindung betrifft in erster Linie Verbindungen der Formel (Ia), worin $R_o$ Wasserstoff oder Niederalkanoyl mit bis und mit 5 C-Atomen, wie Acetyl, darstellt, $R_1$ Hydroxymethyl bedeutet, $R_2$ Niederalkyl mit bis und mit 4 C-Atomen, wie Methyl, darstellt, $R_3$ 5- bis 7-gliedriges Niederalkylenamino, Morpholin-4-yl oder Pyrrol-1-yl ist, $R_a$ und $R_c$ Wasserstoff bedeuten und $R_b$ Halogen bis und mit Atomnummer 35, wie Chlor, oder Niederalkyl mit bis und mit 4 C-Atomen, wie Methyl, darstellt und ihre Salze und Isomeren.

Die Erfindung betrifft in erster Linie Verbindungen der Formel (Ia), worin $R_o$ Wasserstoff oder Niederalkanoyl mit bis und mit 5 C-Atomen, wie Acetyl, dar-

stellt, $R_1$ Formyl bedeutet, $R_2$ Niederalkyl mit bis und mit 4 C-Atomen, wie Methyl, darstellt, $R_3$ 5- bis 7-gliedriges Niederalkylenamino, Morpholin-4-yl oder Pyrrol-1-yl ist, $R_a$ und $R_c$ Wasserstoff bedeuten und $R_b$ Halogen bis und mit Atomnummer 35, wie Chlor, oder Niederalkyl mit bis und mit 4 C-Atomen, wie Methyl, darstellt und ihre Salze und Isomeren.

Die Erfindung betrifft in erster Linie Verbindungen der Formel (Ia), worin $R_o$ Wasserstoff oder Niederalkanoyl mit bis und mit 5 C-Atomen, wie Acetyl, bedeutet, $R_1$ Hydroxymethyl, Niederalkanoyloxymethyl, Niederalkoxymethyl, Formyl oder Diniederalkoxymethyl darstellt, $R_2$ Niederalkyl mit bis und mit 4 C-Atomen, wie Methyl, bedeutet, $R_3$ Pyrrol-1-yl darstellt, $R_a$ und $R_c$ jeweils Wasserstoff bedeuten und $R_b$ Niederalkyl mit bis und mit 4 C-Atomen, wie Methyl, oder Halogen bis und mit Atomnummer 35, wie Chlor, darstellt, und ihre Salze und Isomeren.

Die Erfindung betrifft insbesondere die in den Beispielen genannten neuen Verbindungen, ihre Salze, vor allem pharmazeutisch verwendbare Salze, und Isomeren, ferner die in den Beispielen beschriebenen Verfahren zu ihrer Herstellung.

Die erfindungsgemässen Verbindungen können z.B. in an sich bekannter Weise hergestellt werden, beispielsweise indem man in einer Verbindung der Formel

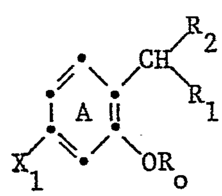

(II) ,

einem Salz oder Isomeren davon, worin $X_1$ für einen in $R_3$ überführbaren Rest steht, $X_1$ in $R_3$ überführt und gewünschtenfalls eine erhaltene

erfindungsgemässe Verbindung in eine andere erfindungsgemässe Verbindung überführt, und/oder gewünschtenfalls ein erhaltenes Salz in die freie erfindungsgemässe Verbindung und/oder in ein anderes erfindungsgemässes Salz überführt und/oder gewünschtenfalls eine erhaltene erfindungsgemässe Verbindung mit salzbildenden Eigenschaften in ein Salz überführt und/oder gewünschtenfalls ein erhaltenes Isomerengemisch in die einzelnen Komponenten auftrennt.

Ein in einen nicht-aromatischen Rest $R_3$ überführbarer Rest $X_1$ bedeutet beispielsweise die Aminogruppe oder eine Gruppe der Formel $-NH-A_1$ bzw. $-NH-A_2-X_2$, wobei $A_1$ dem einwertigen Kohlenwasserstoffrest entspricht, den die durch zwei einwertige Kohlenwasserstoffrest disubstituierte Aminogruppe aufweist, $A_2$ dem zweiwertigen, gegebenenfalls durch mindestens ein Heteroatom unterbrochenen Kohlenwasserstoffrest entspricht, durch den die durch einen zweiwertigen, gegebenenfalls durch mindestens ein Heteroatom unterbrochenem Kohlenwasserstoffrest disubstituierte Aminogruppe $R_3$ disubstituiert ist, und $X_2$ Hydroxy oder insbesondere reaktionsfähiges verestertes Hydroxy darstellt. Dieses kann z.B. Halogen, wie Chlor, Brom oder Iod, Hydroxysulfonyloxy, oder Sulfonyloxy, z.B. gegebenenfalls substituiertes Phenyl- bzw. Niederalkan-sulfonyloxy, wie p-Toluol-, p-Bromphenyl-, Methan- oder Trifluormethan-sulfonyloxy, ferner gegebenenfalls substituiertes Niederalkanoyloxy, wie Acetyl- oder Trifluoracetyloxy, bedeuten.

Die Ueberführung von Amino bzw. $-NH-A_1$ in $R_3$ erfolgt in an sich bekannter Weise. So setzt man beispielsweise eine entsprechende Verbindung der Formel II, ein Salz oder Isomeres davon mit einer Verbindung der Formel $A_1-X_2$ (IIa) oder einem Salz davon um, wobei man je nach Ausgangsmaterial der Formel II ein bzw. mindestens zwei Mol der Verbindung der Formel IIa einsetzt. Dabei arbeitet man in Ab- oder üblicherweise in Anwesenheit eines geeigneten Lösungs- oder Verdünnungsmittels oder eines Gemisches derselben, unter Kühlen, bei Raumtemperatur oder unter Erwärmen, z.B. in einem Temperaturbereich

von etwa -10°C bis etwa 250°C, vorzugsweise bei etwa 20° bis etwa 150°C, gegebenenfalls in einer Inertgasatmophäre, z.B. Stickstoff, und üblicherweise in Gegenwart einer Säure oder insbesondere einer Base. Als Säuren kommen z.B. Protonsäuren, wie Mineralsäuren, z.B. eine Halogenwasserstoffsäure, Schwefel- oder eine Phosphorsäure, oder Sulfonsäuren, wie gegebenenfalls substituierte Phenyl- bzw. Niederalkansulfonsäure, z.B. p-Toluolsulfonsäure, oder gegebenenfalls substituierte Niederalkancarbonsäuren, wie Essigsäure, in Frage. Geeignete Basen sind z.B. Alkalimetall-niederalkanolate, -amide, -hydride, -hydroxide, -carbonate, -alkanoate, gegebenenfalls cyclische und/oder tertiäre Stickstoffbasen, wie Niederalkylaminverbindungen, z.B. Trition B, Triethyl- oder Ethyldiisopropyl-amin, oder cyclische Amine, z.B. Piperidin, Collidin, 1,5-Diaza-bicyclo[4.3.0]non-5-en (DBN) oder 1,8-Diazabicyclo[5.4.0]undec-7-en (DBU), ferner Lithiumdiniederalkylamide, z.B. Lithium-diethyl- oder Lithium-diisopropyl--amid, oder Silanamine, z.B. Hexamethyldisilazan (HMDS).

Zur Ueberführung von Verbindungen der Formel II, worin $X_1$ Amino ist, in Verbindungen der Formel I, worin $R_3$ eine durch einen zweiwertigen, gegebenenfalls durch mindestens ein Heteroatom unterbrochenen Kohlenwasserstoff disubstituierte Aminogruppe bedeutet, kann man eine entsprechende Aminoverbindung der Formel II z.B. mit einer Verbindung der Formel $X_2$-$A_2$-$X_2$ (IIb) umsetzen, wobei man die Umsetzung unter den vorstehend aufgeführten Reaktionsbedingungen durchführen kann. Dabei kann intermediär eine Ausgangsverbindung der Formel II gebildet werden, worin $X_1$ für eine Gruppe der Formel -NH-$A_2$-$X_2$ steht, die unter den Reaktionsbedingungen zu der Zielverbindung weiterreagiert. Entsprechende Ausgangsverbindungen können, brauchen jedoch nicht, isoliert werden.

In einer bevorzugten Ausführungsform zur Herstellung von Verbindungen der Formel I, worin $R_3$ Morpholin-4-yl bzw. Thiomorpholin-4-yl bedeutet, geht man beispielsweise von Ausgangsmaterial der Formel II aus, worin $X_1$ für die Gruppe der Formel $-N(CH_2-CH_2-OH)_2$ bzw. $-N(CH_2-CH_2-SH)_2$ steht, und cyclisiert dieses beispielsweise in Gegenwart einer Protonsäure, wie Schwefelsäure. Ebenso kann man zu derartigen Verbindungen der Formel I gelangen, indem man Ausgangsmaterial der Formel II, worin $X_1$ Amino ist, z.B. mit einem 2,2'-Dihalogen-, wie 2,2'-Dibrom-diethyl(thio)ether in Gegenwart einer Base, z.B. einer der vorstehend genannten, wie Ethyl-diisopropyl-amin, umsetzt. Dabei kann intermediär eine Verbindung der Formel II gebildet werden, worin $X_1$ für die Gruppe der Formel $-NH-C_2H_4-O-C_2H_4-Hal$ ($-NH-A_2-X_2$) bzw. die entsprechende Thioform steht. Zweckmässig wird eine derartige Zwischenverbindung nicht isoliert, sondern unter den Reaktionsbedingungen weiter umgesetzt.

Ein Rest $R_3$ kann, sofern dieser nicht-aromatischer Natur ist, auch direkt eingeführt werden, indem man beispielsweise von einer Verbindung der Formel II und $R_3-X_3$ (IIc) ausgeht, wobei einer der Reste $X_1$ und $X_3$ Wasserstoff und der andere reaktionsfähiges verestertes Hydroxy, z.B. wie vorstehend angegeben, bedeutet. Für den Fall, dass $X_1$ Wasserstoff ist und $X_3$ z.B. für Halogen steht, kann die Umsetzung z.B. in Gegenwart einer Protonsäure oder Lew issäure, z.B. einer Halogenverbindung des Aluminiums, Bors, Zinns(IV), Zinks(II), wie Aluminiumchlorid, durchgefürt werden.

Ebenso können genügend nucleophile Amine $R_3$-H direkt in solche Verbindungen der Formel (II) eingeführt werden, worin $X_1$ für einen durch $R_3$ ersetzbaren Rest steht. Bedeutet beispielsweise $X_1$ Halogen, insbesondere Chlor, Brom oder Iod, kann die Umsetzung in An- oder Abwesenheit eines Lösungsmittels und je nach Wahl des Halogenatoms bei niedrigen Temperaturen bis zur Siedetemperatur des betreffenden Lösungsmittels, zusätzlich vorteilhaft in Gegenwart eines Kupfer(I)-salzes erfolgen. Vorteilhaft befindet sich in der Nachbarposition von $X_1$ ein Substituent mit starkem -I- oder -M-Effekt, wie Nitro, Halogen oder Trifluormethyl. In einzelnen Fällen ist es von Vorteil, die Umsetzung unter Druck oder bei erhöhter Temperatur durchzuführen. Vorteilhaft setzt man die Amine im Ueberschuss ein.

Ebenso können genügend nucleophile Amine $R_3$-H direkt in solche Verbindungen der Formel (II) eingeführt werden, worin $R_o$ sowie $X_1$ jeweils für Wasserstoff stehen. Hierzu werden z.B. entsprechende Verbindungen der Formel (II) zunächst mit einem Oxidationsmittel, wie Blei(IV)acetat, z.B. in Gegenwart einer geeigneten Säure, wie Eisessig, und bei Raumtemperatur, behandelt und anschliessend mit entsprechenden Aminen der Formel $R_3$-H in einem inerten Lösungsmittel, wie einem Ether, z.B. Dioxan, unter Erhitzen, z.B. auf Rückflusstemperatur, umgesetzt.

Werden diese Umsetzungen in Gegenwart einer Base durchgeführt, kann z.B. gegebenenfalls vorhandenes Acyl, wie Niederalkanoyloxy, zu Hydroxy hydrolysiert werden. Hydroxy $OR_o$ bzw. Hydroxymethyl $R_1$ können, sofern erwünscht, anschliessend in der nachstehend angegebenen Weise wieder verestert werden.

Verbindungen der Formel (I), worin $R_3$ Pyrrol-1-yl bedeutet, sind z.B. erhältlich, indem man Verbindungen der Formel (II), worin $X_1$ Amino bedeutet, oder deren Salze mit 2-Buten-1,4-diol oder einem reaktionsfähig veresterten Derivat davon in Gegenwart einer Base, wie Triethylamin, zum Pyrrolin-1-yl umsetzt und dieses in Gegenwart eines Dehydrierungskatalysators, wie eines Chinons, z.B. 2,3-Dichlor-5,6-dicyano-p-benzochinon oder Tetrachlor-p-benzochinon , oder eines Selenderivates, z.B. Selendioxid, oder eines Elements der VIII. Nebengruppe, wie Palladium, dehydriert oder indem man Verbindungen der Formel (II), worin $X_1$ für Amino steht, oder deren Salze mit einem 2,5-Diniederalkoxytetrahydrofuran, wie 2,5-Dimethoxy-tetrahydrofuran, z.B. unter Erwärmen uhd in Gegenwart einer Protonsäure, behandelt.

BAD ORIGINAL

Weiterhin kann der Pyrrolring $R_3$ aufgebaut werden, indem beispielsweise die Aminogruppe $X_1$ in Verbindungen der Formel (II) mit einem gegebenenfalls reaktionsfähigen veresterten Derivat von 1,3-Butadien-1,4-diol, z.B. mit 1,4-Dibrom-1,3-butadien, erforderlichenfalls unter Erwärmen und unter Schutzgas z.B. Stickstoff, und in Gegenwart einer der vorstehend genannten Base sowie vorteilhaft einer aktivierenden Nickelverbindung umgesetzt wird.

Ferner kann der Pyrrolring $R_3$ analog Knorr-Paal durch Behandeln der Aminogruppe $X_1$ in Verbindungen der Formel (II) mit gegebenenfalls acetalisiertem 1,4-Dioxo-butan aufgebaut werden, wobei beispielsweise unter Schutzgas und Erwärmen und in einem inerten Lösungsmittel gearbeitet werden kann.

Eine weitere Verfahrensvariante zum Aufbau des Pyrrolrings $R_3$ besteht z.B. in der Umsetzung von Verbindungen der Formel (II), worin $X_1$ z.B. die Gruppe der Formel -NH-CH=CH-CH=CH-OH bzw. eine reaktionsfähige veresterte Form, ferner eine tautomere Form davon, die gegebenenfalls acetalisiert ist, darstellt. Hierbei wird die Reaktion z.B. vorteilhaft in Gegenwart einer der vorstehend genannten Base durchgeführt.

Eine weitere Verfahrensvariante zur Herstellung von Verbindungen der Formel I besteht darin, dass man z.B. in einer Verbindung der Formel

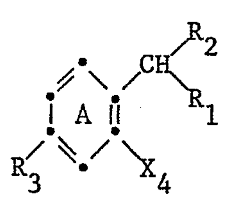

(III) ,

einem Salz oder Isomeren davon, worin $X_4$ für einen in die Gruppe $-OR_o$ überführbaren Rest steht, den Rest $X_4$ in die Gruppe $-OR_o$ überführt und gewünschtenfalls eine erhaltene erfindungsgemässe Verbindung in eine andere erfindungsgemässe Verbindung überführt

und/oder gewünschtenfalls ein erhaltenes Salz in die freie erfindungsgemässe Verbindung oder in ein anderes erfindungsgemässes Salz überführt und/oder gewünschtenfalls eine erhaltene erfindungsgemässe Verbindung mit salzbildenden Eigenschaften in ein Salz überführt und/oder gewünschtenfalls ein erhaltenes Isomerengemisch in die einzelnen Komponenten auftrennt.

Ein in die Gruppe $-OR_o$ überführbarer Rest $X_4$ ist beispielsweise eine Diazoniumgruppe mit einem Anion einer anorganischen oder organischen Säure als Gegenion.

Die Substitution der Diazoniumgruppe durch Hydroxy erfolgt in an sich bekannter Weise, beispielsweise durch Erwärmen, z.B. auf etwa 100° bis etwa 250°C, in wässriger Lösung. Häufig wird diese Umsetzung in Gegenwart von Säuren, wie Mineralsäuren, insbesondere Schwefel- oder Orthophosphorsäure, durchgeführt und als Gegenion wird das Hydrogensulfation bevorzugt. Zur Vermeidung einer Azokupplung wird das gebildete Phenol laufend aus dem Reaktionsgemisch entfernt, beispielsweise durch Ausschütteln mit einem geeigneten Lösungsmittel.

Ein in die Gruppe $OR_o$ überführbarer Rest $X_4$ kann weiterhin z.B. geschütztes Hydroxy, wie verethertes Hydroxy oder von $OR_o$ verschiedenes Acyloxy bedeuten.

Unter verethertem Hydroxy $X_4$ ist z.B. mit einem gegebenenfalls substituierten aliphatischen Alkohol verethertes Hydroxy, beispielsweise Niederalkoxy, wie Methoxy oder tert.-Butyloxy,Aryl, wie mindestens ein gegebenenfalls, z.B. durch Niederalkoxy, substituiertes Phenyl, aufweisendes 1-Niederalkoxy, z.B. Benzyloxy, Triphenylmethoxy oder Dimethoxytriphenyl-methoxy, durch Niederalkoxyniederalkoxy substituiertes 1-Niederalkoxy, z.B. Methoxyethoxy-methoxy, sowie 2-Tetrahydro-pyranyloxy, oder Silyloxy, wie Niederalkyl und/oder Phenyl

aufweisendes Silyloxy, z.B. Trimethyl-, Isopropyl-dimethyl-, tert.-Butyl-diphenyl-silyloxy, zu verstehen.

Von $-OR_o$ verschiedenes Acyloxy $X_4$ leitet sich z.B. von einer Carbonsäure oder Kohlensäure ab. Derartige Reste $X_4$ sind z.B. gegebenenfalls substituiertes Benzoyloxy, gegebenenfalls, z.B. durch Halogen, substituiertes Niederalkanoyloxy, wie Acetyl-, Pivaloyl-, Trichloracetyl- oder Tribromacetyloxy, Formyl-oxy, gegebenenfalls, z.B. durch gegebenenfalls substituiertes Phenyl oder Halogen, substituiertes Niederalkoxycarbonyloxy, wie Ethoxy-, Benzyloxy-, Trichlorethoxy-, Tribromethoxy-carbonyloxy.

Die Ueberführung derartiger Reste $X_4$ in Hydroxy-$OR_o$ erfolgt in an sich bekannter Weise, beispielsweise durch Solvolyse, z.B. Hydrolyse, z.B. in Gegenwart einer Säure oder Base, durch Reduktion, z.B. durch Hydrogenolyse in Gegenwart eines Hydrierungskatalysators oder durch metallische Reduktionssysteme, oder durch Acidolyse. Dabei wird in Ab- oder üblicherweise in Anwesenheit eines geeigneten Lösungs- oder Verdünnungsmittels oder eines Gemisches derselben, unter Kühlen, bei Raumtemperatur oder unter Erwärmen, z.B. in einem Temperaturbereich von etwa -10°C bis etwa 150°C, vorzugsweise bei etwa 0° bis etwa 100°C, gegebenenfalls in einer Inertgasatmospäre, z.B. Stickstoff, und/oder unter Druck, gearbeitet.

Als Säuren kommen beispielsweise Protonsäuren, wie Mineralsäuren, z.B. eine Halogenwasserstoffsäure, Schwefel- oder eine Phosphorsäure, oder Sulfonsäuren, wie gegebenenfalls substituierte Phenyl- bzw. Niederalkansulfonsäure, z.B. p-Toluolsulfonsäure, oder gegebenenfalls subsituierte Niederalkancarbonsäure, z.B. Essigsäure, in Frage. Bei einigen Reaktionsweisen kann die Spaltung z.B. in Gegenwart einer Lewissäure, z.B. einer Halogenverbindung des Aluminiums, Bors, Zinns (IV), Zinks (II), wie Aluminiumchlorid, Bortrifluorid oder

Bortribromid, erfolgen. Geeignete Basen sind z.B. Alkalimetall-niederalkanolate, -amide, -hydride, -hydroxide, -carbonate, -niederalkanoate, gegebenenfalls cyclische und/oder tertiäre Stickstoffbasen, wie Niederalkylaminverbindungen, z.B. Triton B, Triethyl- oder Ethyldiisopropyl-amin, oder cyclische Amine, z.B. Piperidin, Collidin, 1,5-Diaza-bicyclo[4.3.0]-non-5-en (DBN) oder 1,8-Diaza-bicyclo[5.4.0]undec-7-en (DBU), ferner Lithiumdiniederalkylamide, z.B. Lithium-diethyl- oder Lithium-diisopropyl-amid, oder Silanamine, z.B. Hexamethyldisilazan (HMDS).

Als Hydrierungskatalysatoren kommen z.B. Elemente der VIII-Nebengruppe des Periodensystems oder ein Derivat, z.B. Oxid, davon in Betracht, wobei der Katalysator gegebenenfalls auf einem geeigneten Trägermaterial, wie Aktivkohle oder Erdalkalimetallcarbonat bzw. -sulfat, aufgezogen sein kann. Als Beispiele für derartige Katalysatoren sind z.B. Raney-Nickel, Cobalt, Rhodium, Palladium auf Kohle, Platin oder Plationoxid zu nennen.

Ein metallisches Reduktionssystem besteht z.B. aus einem (unedlen), Metall, wie Alkali- oder Erdalkalimetallsalz, z.B. Natrium, Lithium, Magnesium oder Calcium, oder Uebergangsmetallsalz, z.B. Zink, Zinn, Eisen oder Titan, und aus einem protonenabspaltenden Mittel, wie Protonsäure, z.B. Salz- oder Essigsäure, Niederalkanol, z.B. Ethanol, oder flüssigem Ammoniak. Solche Systeme sind beispielsweise Natrium/Ammoniak, Zink/Salz- oder Essigsäure oder Zink/Ethanol.

In bevorzugten Ausführungsformen kann z.B. Niederalkoxy $X_4$ in Gegenwart einer Halogenwasserstoffsäure, die gegebenenfalls in Form eines Pyridiniumsalzes vorliegen kann, z.B. Bromwasserstoffsäure oder Pyridiniumchlorid, oder einer Lewissäure zu Hydroxy $-OR_o$ gespalten werden. 1-Phenylniederalkoxy, wie Benzyloxy, kann z.B. durch Hydrogenolyse in Gegenwart eines Hydrierungskatalysators, ein Tritylrest bzw. 2-Tetrahydropyranyloxy kann vorteilhaft in Gegenwart einer Protonsäure,

z.B. Essigsäure, und einer der bezeichneten Silylether in Gegenwart einer Protonsäure, z.B. Essigsäure, bzw. eines Fluorids, z.B. Tetrabutylammoniumfluorid, zu Hydroxy gespalten werden. Benzoyl sowie Niederalkanoyl können vorteilhaft in Gegenwart einer Base, wie Natronlauge, zu Hydroxy hydrolysiert werden, während z.B. Trichloracetoxy (z.B. durch Zn-Cu in Essigsäure), Trichlorethoxycarbonyloxy (z.B. durch Zn/Salzsäure) und Benzyloxycarbonyloxy (z.B. durch Hydrogenolyse in Gegenwart eines Hydrierungskatalysators) reduktiv in Hydroxy gespalten werden können.

Unter diesen Reaktionsbedingungen dieser Verfahrensvariante kann verethertes oder verestertes Hydroxymethyl bzw. acetalisiertes Formyl gleichzeitig in Hydroxymethyl bzw. Formyl überführt und Alkoxyreste des Rings A gespalten werden.

Ein anderes Herstellungsverfahren für Verbindungen der Formel I besteht darin, dass beispielsweise in einer Verbindung der Formel

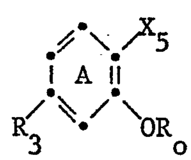

(IV)

oder einem Salz davon, worin $X_5$ für einen in die Gruppe der Formel $-CH(R_2)-R_1$ überführbaren Rest steht, $X_5$ in die Gruppe der Formel $-CH(R_2)-R_1$ überführt und gewünschtenfalls eine erhaltene erfindungsgemässe Verbindung in eine andere erfindungsgemässe Verbindung überführt und/oder gewünschtenfalls ein erhaltenes Salz in die freie erfindungsgemässe Verbindung oder in ein anderes erfindungsgemässes Salz überführt und/oder gewünschtenfalls eine erhaltene erfindungsgemässe Verbindung mit salzbildenden Eigenschaften in ein Salz überführt und/oder gewünschtenfalls ein erhaltenes Isomerengemisch in die einzelnen Komponenten auftrennt.

Der Rest $X_5$ bedeutet beispielsweise eine Gruppe der Formel $-CH(R_2)-Y_1$, in der $Y_1$ z.B. für folgende Bedeutungen steht: gegebenenfalls funktionell abgewandeltes Carboxy, geschütztes bzw. maskiertes Hydroxymethyl bzw. Formyl, eine Gruppe der Formel $-CH_2-Y_2$, in der $Y_2$ reaktionsfähiges verestertes Hydroxy, darstellt, die Gruppe der Formel -CO-COOH oder Gruppen der Formeln $-CH(OH)-CH(OH)-Y_3$ bzw. $-CH=CH-Y_3$, in denen $Y_3$ jeweils Wasserstoff, ein aliphatischer Rest, z.B. gegebenenfalls substituierter Niederalkylrest, oder ein Arylrest bedeutet und unter diesem z.B. ein gegebenenfalls substituierter Phenylrest zu verstehen ist. Der Rest $X_5$ steht ebenfalls für eine Gruppe der Formel 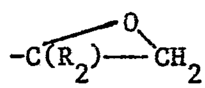 , für eine Gruppe der Formel $-C(R_2)=CH-Y_4$, wobei $Y_4$ für Wasserstoff oder verethertes Hydroxy steht, sowie für eine Gruppe $-Me-Y_2$ bzw. $-Y_2$, wobei Me z.B. für ein zweiwertiges Metall, wie Magnesium oder Zink, steht.

Die nachfolgend beschriebenen Umsetzungen werden in an sich bekannter Weise durchgeführt, sofern nicht abweichend aufgeführt, in Ab- oder üblicherweise in Anwesenheit eines geeigneten Lösungs- oder Verdünnungsmittels oder eines Gemisches derselben, wobei man unter Kühlen, bei Raumtemperaturen oder unter Erwärmen, z.B. in einem Temperaturbereich von etwa -80° bis etwa 250°, vorzugsweise bei Raumtemperatur bis zum Siedepunkt des betreffenden Lösungsmittels, und, falls erforderlich, in einem abgeschlossenen Gefäss, unter Druck, in einer Inertgasatmosphäre, z.B. unter Stickstoff, und/oder unter wasserfreien Bedingungen arbeitet.

In Verbindungen der Formel IV, worin $X_5$ für die Gruppe der Formel $-CH(R_2)-Y_1$ und $Y_1$ gegebenenfalls funktionell abgewandeltes Carboxy steht, kann $Y_1$ z.B. reduktiv in $R_1$ Hydroxymethyl bzw. Formyl übergeführt werden.

Funktionell abgewandeltes Carboxy ist beispielsweise verestertes, amidiertes oder anhydridisiertes Carboxy oder Cyano. Verestertes Carboxy ist beispielsweise mit einem aliphatischen, cycloaliphatischen oder aromatischen Alkohol verestertes Carboxy, beispielsweise gegebenenfalls, z.B. durch Hydroxy, Niederalkoxy, Niederalkanoyloxy, Halogen oder gegebenenfalls Substituenten aufweisendes Phenyl, substituiertes Niederalkoxycarbonyl, Cycloniederalkyloxycarbonyl oder gegebenenfalls, z.B. ein- oder mehrfach, substituiertes Phenoxycarbonyl. Amidiertes Carboxy ist z.B. Carbamoyl oder z.B. durch gegebenenfalls substituierte aliphatische oder aromatische Reste ein- oder zweifach substituiertes Carbamoyl, beispielsweise Mono- oder Diniederalkylcarbamoyl, Mono- oder Di-phenylniederalkyl-carbamoyl, Mono- oder Di-phenyl-carbamoyl, Niederalkylencarbamoyl, ferner Niederalkenylencarbamoyl. Anhydridisiertes Carboxy ist beispielsweise mit einer Halogenwasserstoffsäure oder einer Carbonsäure, wie einer gegebenenfalls substituierten Niederalkancarbonsäure bzw. Benzoesäure oder einem Kohlensäurehalogenid-niederalkylhalbesters, anhydridisiertes Carboxy, beispielsweise Halogen-, wie Chlorcarbonyl, Niederalkanoyloxycarbonyl, wie Acetyloxycarbonyl, oder Niederalkoxycarbonyloxycarbonyl, wie Ethoxycarbonyloxy.

Je nach Wahl der Reaktionsbedingungen und Reduktionsmittel kann die Reduktion zu solchen Verbindungen der Formel I führen, worin $R_1$ Hydroxymethyl oder Formyl darstellt. Insbesondere unter milden Reaktionsbedingungen gelangt man zu den entsprechenden Formylverbindungen der Formel I oder I'.

Derartige Reste $Y_1$ können in an sich bekannter Weise zu Hydroxymethyl bzw. Formyl $R_1$ reduziert werden, beispielsweise unter Verwendung von Wasserstoff in Gegenwart eines Hydrierungskatalysators oder von Hydrid- übertragenden Reagentien, wobei gegebenenfalls zusätzlich

Lewissäuren verwendet werden können. Ferner kann Di-alkalimetall-tetra-carbonyl-ferrat (z.B. $Na_2Fe(CO)_4$) als Reduktionsmittel verwendet werden. Als Hydrierungskatalysator kommt z.B. ein Element der VIII. Nebengruppe des Periodensystems oder ein Derivat, z.B. Oxid, davon in Betracht, wobei der Katalysator gegebenenfalls auf einem geeigneten Trägermaterial, wie Aktivkohle oder Erdalkalimetallcarbonat bzw. -sulfat, aufgezogen sein kann. Als Beispiele für derartige Katalysatoren sind z.B. Raney-Nickel, Cobalt, Rhodium, Palladium auf Kohle, Platin oder Platinoxid zu nennen. Geeignete Hydrid-übertragende Reagentien sind beispielsweise Hydride, z.B. solche mit Elementen der 3. Hauptgruppe, wie Diboran oder Diisobutylaluminiumhydrid, komplexe Hydride, z.B. solche mit Elementen der 1. und 3. Hauptgruppe des Periodensystems, wie Lithiumaluminiumhydrid oder Natriumborhydrid, gegebenenfalls in Kombination mit einer Lewissäure, wie Aluminiumchlorid oder Bortrifluorid, Alkalimetall-triniederalkoxy-aluminiumhydride, z.B. Lithium-tri-tert.-butyloxy- oder Lithium-triethoxy--aluminiumhydrid, sowie weitere Hydride, z.B. Tributylzinnhydrid.

Aus Verbindungen der Formel IV, worin $X_5$ für eine Gruppe der Formel $-CH(R_2)-COOH$ steht und $R_o$ Wasserstoff bedeutet, gebildete cyclische Ester, d.h. Lactone der Formel

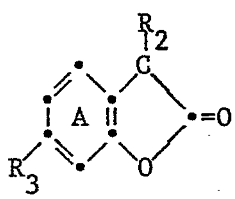

(IV') ,

können ebenfalls reduktiv in die entsprechenden Hydroxymethyl- bzw. Formyl-Verbindungen der Formel I übergeführt werden.

Verestertes Carboxy $Y_1$ kann weiterhin, z.B. mit Hilfe von Trihalogen-, wie Trichlorsilan oder eines geeigneten Hydrid-übertragenden Reagenz, wie mit Diboran, welches vorteilhaft aus Natriumborhydrid und Bortrifluorid-Etherat gebildet werden kann, und Niederalkoxythiocarbonyl kann z.B. durch Hydrierung, z.B. in Gegenwart von Raney-Nickel zu veretherten Hydroxymethyl $R_1$ reduziert werden.

Steht der Rest $X_5$ z.B. für eine Gruppe des Formel $-CH(R_2)-Y_1$, in der $Y_1$ geschütztes oder maskiertes Hydroxymethyl bzw. Formyl bedeutet, kann $Y_1$ in an sich bekannter Weise in Hydroxymethyl bzw. Formyl $R_1$ übergeführt werden.

In geschütztem Hydroxymethyl $Y_1$ kann die Hydroxygruppe beispielsweise als mit einem gegebenenfalls substituierten aliphatischen Alkohol verethertes Hydroxy, als 2-Tetrahydropyranyloxy oder als Silyloxy sowie als mit einer Carbonsäure oder mit einer Kohlensäure verestertes Hydroxy vorliegen. Als mit einem gegebenenfalls substituierten aliphatischen Alkohol verestertes Hydroxy kommt z.B. Niederalkoxy, wie Methoxy oder tert.-Butyloxy, Aryl, wie mindestens ein gegebenenfalls, z.B. durch Niederalkoxy, substituiertes Phenyl, aufweisendes 1-Niederalkoxy, z.B. Benzyloxy, Triphenylmethoxy- oder Dimethoxytriphenyl-methoxy, durch Niederalkoxyniederalkoxy substituiertes Niederalkoxy, z.B. Methoxyethoxy-methoxy, und als Silyloxy z.B. Niederalkyl und/oder Phenyl aufweisendes Silyloxy, wie Trimethyl-, Isopropyl-dimethyl-, tert.-Butyl-dimethyl- oder tert.-Butyl-diphenyl-silyloxy, in Frage. Mit einer Carbonsäure bzw. Kohlensäure verestertes Hydroxy stellt beispielsweise gegebenenfalls substituiertes Benzoyloxy, gegebenenfalls, z.B. durch Halogen, substituiertes Niederalkanoyloxy, wie Acetyl-, Pivaloyl-, Trichloracetyl- oder Tribromacetyloxy, Formyloxy, bzw. gegebenenfalls, z.B. durch gegebenenfalls substituiertes Phenyl oder Halogen, substituiertes Niederalkoxycarbonyloxy, wie Ethoxy-, Benzyloxy-, Trichlorethoxy- oder Tribromethoxy-carbonyloxy, dar.

Die Ueberführung derartiger Reste $Y_1$ in Hydroxymethyl $R_1$ erfolgt in Analogie zu der Ueberführung von geschütztem Hydroxy $X_4$ der vorstehenden Verfahrensvariante. Dabei kann verestertes Hydroxy-$OR_o$ gleichzeitig in Hydroxy-$OR_o$ übergeführt werden.

Geschütztes Formyl $Y_1$ bedeutet beispielsweise thioacetalisiertes Formyl, gegebenenfalls substituiertes Iminomethyl oder eine entsprechende tautomere Enaminform davon ein 2-Dihydro-oxazinyl-, 2-Oxazolidinyl- oder 2-Thiazolidinylrest oder 2,2-Dicyano-ethenyl. Thioacetalisiertes Formyl ist z.B. Niederalkoxyniederalkylthio-methyl, Diniederalkylthio-methyl, Oxyniederalkylenthio- oder Niederalkylendithio-methyl, beispielsweise Ethoxy-ethylthio-, Diethylthio-, 1-Oxy-ethylen-2-thio-, Ethylendithio- oder 1,3-Propylendithio-methyl. Als Substituenten von substituiertem Iminomethyl kommen z.B. gegebenenfalls substituierte Kohlenwasserstoffreste, wie entsprechende aliphatische oder aromatische Reste, z.B. gegebenenfalls Phenyl aufweisendes Niederalkyl, beispielsweise Ethyl oder Benzyl, oder eine von einer Carbonsäure oder einem Halbester oder Kohlensäure abgeleitete Acylgruppe, wie Niederalkanoyl, gegebenenfalls substituiertes Benzoyl, oder gegebenenfalls substituiertes Niederalkoxycarbonyl, ferner 2-Benzothiazolyl in Frage.Ein 2-Dihydro-oxazinylrest ist beispielsweise 4,4,6,6-Tetramethyl- oder 4,4,6-Trimethyl-2,3-dihydro-oxazinyl, und 2-Oxazolidinyl ist z.B. 4,4-Dimethyloxazolidin-2-yl.

Geschütztes Formyl $Y_1$ kann in an sich bekannter Weise, z.B. durch Hydrolyse, gegebenenfalls in Gegenwart eines katalytischen Mittels, in Formyl $R_1$ umgewandelt werden.

Die Hydrolyse kann beispielsweise durch eine Base (z.B. bei 2,2-Dicyanoethenyl) oder eine Säure (z.B. bei thioacetalisiertem Formyl, gegebenenfalls substituiertem Iminomethyl, einer entsprechenden tautomeren Enaminform davon oder einem 2-Oxazolidinyl- oder 2-Thiazolidinylrest) katalysiert werden, wobei die Hydrolyse von thioacetalisiertem Formyl vorteilhaft auch in Gegenwart von Quecksilber-(II)-salzen, gegebenenfalls in Gegenwart einer Lewissäure, z.B. Bortrifluorid, oxidativ mit N-Halogen-succinimiden oder Chloramin-T, ferner mit einem Halogen-triniederalkyl-, wie Brom(Iod)trimethylsilan in Kombination mit Dimethylsulfoxid oder durch Lithium induzierte Hydrolyse, z.B. mit Lithium-triisopropylamin, unterstützt werden kann.

Geeignete Basen sind z.B. Alkalimetall-niederalkanolate, -amide, -hydride, -hydroxide, -carbonate, -alkanoate, gegebenenfalls cyclische und/oder tertiäre Stickstoffbasen, wie Niederalkylaminverbindungen, z.B. Triton B, Triethyl- oder Ethyldiisopropyl-amin, oder cyclische Amine, z.B. Piperidin, Collidin, 1,5-Diaza-bicyclo[4.3.0]non-5-en (DBN) oder 1,8-Diazabicyclo[5.4.0]undec-7-en (DBU), ferner Lithiumdiniederalkylamide, z.B. Lithium-diethyl- oder Lithium-diisopropyl--amid, oder Silanamine, z.B. Hexamethyldisilazan (HMDS).

Als Säuren kommen z.B. Protonsäuren, wie Mineralsäuren, z.B. eine Halogenwasserstoffsäure, Schwefel- oder eine Phosphorsäure, oder Sulfonsäuren, wie gegebenenfalls substituierte Phenyl- bzw. Niederalkansulfonsäure, z.B. p-Toluolsulfonsäure, oder gegebenenfalls substituierte Niederalkancarbonsäuren, wie Essigsäure, in Frage.

In Verbindungen der Formel IV, worin $X_5$ für eine Gruppe der Formel $-CH(R_2)-Y_1$ und $Y_1$ für eine Gruppe der Formel $-CH_2-Y_2$ steht, kann reaktionsfähiges verestertes Hydroxy durch verethertes Hydroxy bzw. verestertes Hydroxy, welches dem verethertem bzw. verestertem Hydroxymethyl $R_1$ enspricht, ersetzt werden.

Derartige Verbindungen der Formel IV können dementsprechend mit einem entsprechenden Alkohol bzw. einer entsprechenden Carbonsäure oder einem Salz davon, insbesondere in Gegenwart einer der vorstehend genannten Base, umgesetzt werden. Die Umsetzung kann vorteilhaft z.B. durch Zusatz eines Kronenethers, z.B. von [18]-Krone-6, beschleunigt werden.

Die Gruppe $-CH_2-Y_2$ kann auch oxidativ in Formyl $R_1$ übergeführt werden, wobei als Oxidationsmittel beispielsweise Dimethylsulfoxid (analog Kornblum), z.B. in Gegenwart einer der vorstehend genannten Base, vorteilhaft unter Zusatz eines Silber(I)salzes, verwendet kann.

Der Rest $Y_1$ kann auch für die Gruppe der Formel -CO-COOH stehen. Diese kann in an sich bekannter Weise zu Formyl $R_1$ decarboxyliert werden. Die Decarboxylierung wird beispielsweise unter Erhitzen, beispielsweise in einem Temperaturbereich von etwa 100° bis etwa 300°C, gegebenenfalls in Gegenwart eines Uebergangsmetalles oder einer Legierung davon, z.B. Kupfer oder Kupferbronze, oder eines Amins, wie eines basischen Stickstoffheterocyclus, z.B. Pyridin oder Chinolin, oder eines Alkylamins, wie Triniederalkylamins, durchgeführt und führt zu Verbindungen der Formel (I), worin $R_1$ Formyl darstellt.

In Verbindungen der Formel IV, worin $X_5$ für eine Gruppe der Formel $-CH(R_2)-Y_1$ und $Y_1$ für Gruppen der Formel $-CH(OH)-CH(OH)-Y_3$ bzw. $-CH=CH-Y_3$ stehen, kann $Y_1$ durch oxidative Spaltung, z.B. durch Oxidation de Diolgruppierung mit Periodsäure oder einem Salz davon oder mit Pyridiniumchlorchromat oder durch Ozonolyse der Doppelbindung, in die Formylgruppe $R_1$ übergeführt werden.

$X_5$ der Formel IV kann weiterhin für eine Gruppe der Formel $-C(R_2)=CH-Y_5$ stehen, wobei $Y_5$ z.B. Wasserstoff, verethertes Hydroxy oder verestertes Hydroxy bedeutet.

An die Gruppe der Formel $-C(R_2)=CH-Y_5$, in der $Y_5$ Wasserstoff bedeutet, kann beispielsweise in an sich bekannter Weise, Wasser angelagert werden, z.B. durch Hydroborierung mit Diboran in Gegenwart einer der vorstehend genannten Basen, wobei solche Verbindungen der Formel I erhältlich sind, worin $R_1$ Hydroxymethyl bedeutet.

Durch Reduktion, z.B. Hydrierung in Gegenwart eines der genannten Hydrierungskatalysators, kann die Gruppe der Formel $-C(R_2)=CH-Y_5$, in der $Y_5$ verethertes Hydroxy bedeutet, in solche Verbindung der Formel I übergeführt werden, worin $R_1$ für verethertes Hydroxymethyl steht.

Zu Verbindungen der Formel I, worin $R_1$ für acetalisiertes Formyl steht, kann man gelangen, indem man z.B. in Ausgangsverbindungen der Formel IV, worin $X_5$ für eine Gruppe der Formel $-C(R_2)=CH-Y_5$ und $Y_5$ für verethertes Hydroxy stehen, an die Gruppe $X_5$ z.B. einen dem veretherten Hydroxymethyl $R_1$ entsprechenden Alkohol, welcher z.B. von einem Orthoameisensäureester abgeleitet sein kann, addiert. Dabei wird üblicherweise in Gegenwart einer der vorstehend genannten Protonsäure oder, bei der Umsetzung mit einem Orthoameisensäureester, Lewissäure gearbeitet.

In Verbindungen der Formel IV kann $X_5$ ebenfalls für die Gruppe der Formel 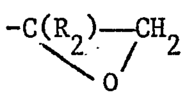 stehen. Durch Behandeln mit einer Lewissäure kann der Oxiranring geöffnet werden, wobei solche Verbindungen der Formel I erhalten werden können, in der $R_1$ Formyl bedeutet. Durch Umsetzung mit einer Lewissäure in Kombination mit einem Reduktionsmittel, wie einem der vorstehend genannten Hydrid-übertragenden Reagenz, einem der genannten reduzierenden metallischen Systeme oder durch Hydrogenolyse in Gegenwart eines Hydrierungskatalysators, können aus entsprechenden, den Oxiranring aufweisenden Ausgangsstoffen der Formel IV solche Verbindungen der Formel I resultieren, worin $R_1$ Hydroxymethyl bedeutet.

Ausserdem kann $X_5$ der Formel IV für eine Gruppe der Formel -Me-Hal oder -Hal stehen, wobei Hal Halogen, wie Chlor, Brom oder Iod , darstellt. Die Ueberführung derartige Ausgangsverbindungen in Verbindungen der Formel I kann in an sich bekannter Weise, z.B. in Analogie zur Grignard-Reaktion, erfolgen.

Bedeutet $X_5$ z.B. -Me-Hal kann die Ueberführung von $X_5$ zur Gruppe der Formel $-CH(R_2)-R_1$ z.B. durch Umsetzung mit einerseits einer Verbindung der Formel $Hal-CH(R_2)-R_1$ oder mit einer Verbindung der Formel

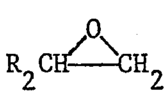 , wobei $R_2$ Wasserstoff ist, durchgeführt werden. Daraus resultieren in letzterem Fall Verbindungen der Formel I, worin $R_1$ Hydroxymethyl ist.

Bedeutet $X_5$ z.B. Hal, können die entsprechenden Ausgangsverbindungen der Formel IV z.B. durch Behandeln mit Verbindungen der Formel Hal-Me-$CH(R_2)$-$R_1$ zu entsprechenden Verbindungen der Formel I umgesetzt werden.

Diese Umsetzungen können vorteilhaft unter Kühlen in einem inerten Lösungs- oder Verdünnungsmittel oder einem Gemisch derselben, gegebenenfalls unter Schutzgas, üblicherweise bei Temperaturen von etwa -80°C bis etwa der Siedetemperatur des Lösungsmittels, erfolgen.

Die Formylgruppe $R_1$ kann vorteilhaft im Verlauf von Oxidationsreaktionen in situ gebildet oder aus einer funktionell abgewandelten Form in Freiheit gesetzt werden. Die in-situ-Bildung von Formyl erfolgt insbesondere aus solchen Resten $X_5$, die in erster Linie Hydroxymethyl oder Gruppen der Formeln $-CH=CH-Y_3$ $-CH(OH)-CH(OH)-Y_3$ oder $-CH(OH)-CO-Y_3$, ferner $-CH=C(Ar)_2$, $-CO-CO-Y_3$ $-CH(OH)-CO-OY_3$ oder $-CH(NH_2)-CO-Y_3$ bedeuten. Die Freisetzung der Formylgruppe $R_1$ erfolgt z.B. aus einem ihrer Acetale oder Imine sowie aus sonstigen Formylmaskierungsgruppen. Acetalisiertes Formyl bedeutet z.B. mit Niederalkanolen oder einem Niederalkandiol acetalisiertes Formyl, wie Diniederalkoxy-, z.B. Dimethoxy- oder Diethoxymethyl, oder Niederalkylendioxy-, z.B. Ethylen- oder Trimethylendioxy-methyl. Ferner kann Formyl aus den entsprechenden Thioacetalen freigesetzt werden. Imine sind z.B. gegebenenfalls substituierte N-Benzylimine oder N-(2-Benzothiazolyl)-imin.

In Verbindungen der Formel

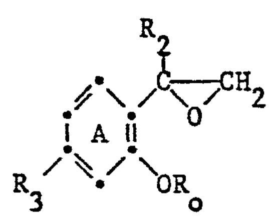

(IV")

wird der Oxiran-Ring, beispielsweise in

Gegenwart einer Lewissäure, wie Aluminiumchlorid, zur Verbindung der Formel (I) geöffnet, worin $R_1$ Formyl darstellt. In fakultativen Zusatzreaktionen kann das Formyl gewünschtenfalls in an sich bekannter Weise acetalisiert oder zu Hydroxymethyl reduziert werden. Die Hydroxymethylgruppe wiederum kann, wenn erwünscht, verestert oder verethert werden.

Die Bereitstellung der Ausgangsverbindungen der Formeln II, III und IV erfolgt z.B. in an sich bekannter Weise.

Als beispielhafte Schlüsselverbindungen für die Herstellung von Ausgangsverbindungen der Formeln II, III und IV sind Verbindungen der Formeln

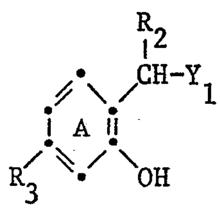 (Va) und 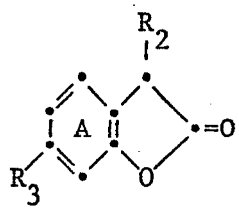 (IV')

anzusehen, worin $Y_1$ z.B. für gegebenenfalls funktionell abgewandeltes Carboxy steht. Zu deren Herstellung kann man beispielsweise von einer Verbindung der Formel

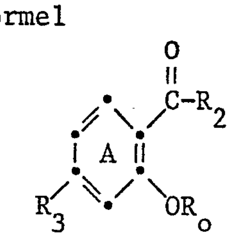 (Vc)

ausgehen, die z.B. durch übliche Friedel-Crafts-Acylierung einer Verbindung der Formel

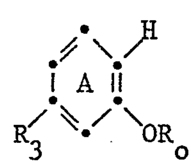 (Vd)

mit einem reaktionsfähiges Säurederivat der Carbonsäure der Formel $R_2$-COOH (Ve), z.B. einem Säurehalogenid davon, erhältlich ist. Dabei

kann vorteilhaft die OH-Gruppe vorübergehend in geschützter Form vorliegen. So kann in einer Verbindung der Formel Vd, worin $R_o$ Wasserstoff ist, die OH-Gruppe $-OR_o$ zweckmässig geschützt werden, wie durch Veretherung, z.B. mit Dimethylsulfat. Im Anschluss an die im nächsten Reaktionsschritt erfolgende Reduktion der Oxo- zur Hydroxy-Gruppe, z.B. durch Behandeln mit einem Hydrid-übertragenden Reagenz, wie Natriumborhydrid, wird zunächst die OH-Gruppe durch Halogen substituiert, z.B. durch Halogenierung mit Thionylchlorid, dann mit Magnesium die entsprechende Magnesium-Halogen-Verbindung hergestellt und diese analog der Grignard-Reaktion mit Kohlendioxid umgesetzt. Daraus resultiert z.B. eine Verbindung der Formel Va, worin $Y_1$ Carboxy ist und die phenolische OH-Gruppe $-OR_o$ gegebenenfalls in geschützter Form vorliegt. Nach Abspaltung der OH-Schutzgruppe, z.B. durch Spaltung eines Ethers mit Bromwasserstoffsäure, kann die resultierende Verbindung der Formel Va z.B. durch Umsetzung mit einem Kondensationsmittel, wie Dicyclohexylcarbodiimid, in eine entsprechende Verbindung der Formel IV' übergeführt werden. Gleichfalls kann in einer Verbindung der Formel Va $Y_1$ in gewünschter Form funktionell abgewandelt und gegebenenfalls die phenolische OH-Gruppe $-OR_o$ in bekannter Weise acyliert werden.

Eine alternative Verfahrensweise zur Herstellung von Verbindungen der Formeln Va und IV', worin $R_2$ Methyl ist, die freien Positionen des Rings A Wasserstoff oder Niederalkyl bzw. die beiden benachbarten freien Positionen gemeinsam 3- bis 4-gliedriges Alkylen darstellen und die dritte freie Position Wasserstoff oder Niederalkyl aufweisen, besteht darin, dass man Verbindungen der Formel

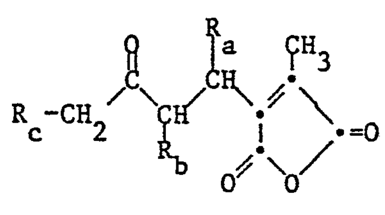

(Vf) ,

worin $R_a$, $R_b$ und $R_c$ unabhängig voneinander Wasserstoff, Niederalkyl oder 3- oder 4-gliedriges Alkylen bedeuten, mit Aminen der Formel $R_3$-H oder deren Säureadditionssalzen umsetzt.

Die Umsetzung erfolgt beispielsweise bei erhöhter Temperatur, z.B. in der Schmelze oder bei der Rückflusstemperatur des Lösungsmittels, z.B. in einem Temperaturbereich von etwa 80° C bis etwa 200° C. Geeignete inerte Lösungsmittel sind beispielsweise höher siedende Kohlenwasserstoffe, wie aromatische Kohlenwasserstoffe, z.B. Benzol, Toluol oder Xylole. Die Amine der Formel $R_3$-H werden insbesondere als Säureadditionssalze, z.B. vorteilhaft als Benzoate, eingesetzt.

Zur Herstellung von Verbindungen der Formel (Vf), worin $R_a$ Wasserstoff bedeutet, geht man von Verbindungen der Formel

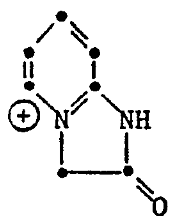 $A^{\ominus}$ (Vg)

aus, die gegebenenfalls im aromatischen Teil substituiert sind und worin $A^{\ominus}$ das Anion einer anorganischen oder organischen Säure darstellt, und setzt diese mit Fumarsäure, Maleinsäure oder Maleinsäureanhydrid in Gegenwart einer Base um, wobei als Basen anorganische oder organische Basen in Frage kommen. Anorganische Basen sind beispielsweise Alkalimetallhydroxide oder -hydride, wie Natrium- oder Kaliumhydroxid oder Natrium- oder Kaliumhydrid. Als organische Amine werden beispielsweise tertiäre Amine, wie Trialkylamine, z.B. Triethylamine oder Tri-n-butylamine, oder cyclische Amine, wie Pyridin, Picolin, Chinolin oder Lutidin, verwendet.

Die auf diesem Wege zunächst erhältlichen freien Verbindungen werden durch Behandeln mit organischen oder anorganischen Säuren in die Salze der Formel

BAD ORIGINAL

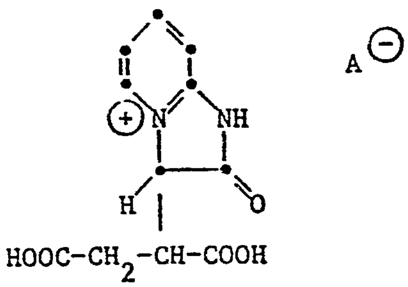

(Vh) ,

übergeführt. Diese werden im weiteren Reaktionsverlauf, gegebenenfalls in Gegenwart einer der vorstehend genannten Basen, mit Verbindungen der Formel $R_a-CH = C(R_b)-CO-CH_2-R_c$ (Vi) zu Verbindungen der Formel

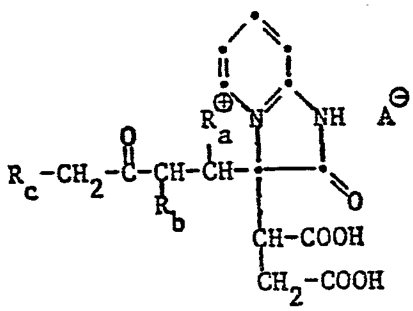

(Vk)

umgesetzt, die im nächsten Reaktionsschritt durch Erhitzen, z.B. auf Temperaturen zwischen 80 und 160°C, unter Decarboxylierung in Verbindungen der Formel

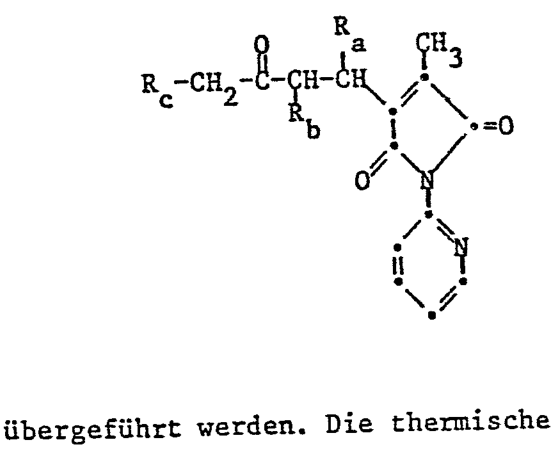

(V 1)

übergeführt werden. Die thermische Ueberführung von Verbindungen der Formel (Vk) in Verbindungen der Formel (V 1) wird beispielsweise in einem gegebenenfalls halogenierten aromatischen Lösungsmittel, wie Benzol, Toluol, Xylol oder Chlorbenzol, oder einer Niederalkancarbonsäure, z.B. Eisessig, durchgeführt. Anschliessend werden die Verbindungen der Formel (V l) zu Verbindungen der Formel (Vf) hydrolysiert. Die Hydrolyse wird beispielsweise in wässrigem oder wässrig-organischem Medium vorgenommen. Als organische Lösungsmittel eignen sich vor allem hochsiedende polare Lösungsmittel, wie Ether, z.B. Dioxan oder Tetrahydrofuran, N,N-Dialkylamide, z.B. N,N-Dimethyl-formamid oder N,N-Dimethylacetamid, oder cyclische Amide, wie N-Methyl-pyrrolidon. Die Hydrolyse erfolgt beispielsweise mit Hilfe einer anorganischen oder organischen Säure, wobei als anorganische Säuren Mineralsäuren, wie Halogenwasserstoffsäure oder Schwefelsäure, und als organische Säuren Sulfonsäuren, wie Niederalkan- oder gegebenenfalls substituierte Benzolsulfonsäuren, wie Methan- oder p-Toluolsulfonsäure, oder gegebenenfalls substituierte Alkancarbonsäuren, wie Eisessig, in Betracht kommen.

Zur Herstellung von Verbindungen der Formel (Vf), worin $R_a$ von Wasserstoff verschieden ist, geht man von Verbindungen der Formel (Vg) aus und setzt diese zunächst mit Verbindungen der Formel (Vi) und anschliessend mit Fumarsäure, Maleinsäure oder insbesondere mit Maleinsäureanhydrid zu Verbindungen der Formel (Vk) um, die wiederum, wie vorstehend beschrieben, weiter zu den entsprechenden Verbindungen der Formel (Vf) weiterreagieren.

Bedeutet $R_3$ der Formel Va bzw. IV' z.B. Dibenzylamino, können die Benzylgruppen unter Bildung der freien Aminogruppe $X_1$ z.B. durch Hydrogenolyse in Gegenwart eines Hydrierungskatalysators abgespalten werden, und $Y_1$ durch Reduktion, z.B. mit einem geeigneten Hydrid-übertragenden Reagenz, in $R_1$ übergeführt werden. In so erhältlichen Hydroxymethyl- bzw. Formyl-Verbindungen kann die Hydroxymethylgruppe $R_1$ gewünschtenfalls verestert oder verethert werden, wobei ebenso auch die phenolische Hydroxygruppe entsprechend verestert oder verethert werden kann, während die Formylgruppe $R_1$ in gewünschter Form acetalisiert werden kann. Es resultieren somit Ausgangsverbindungen der Formel II, worin $X_1$ Amino ist. Dieses kann gewünschtenfalls, z.B. durch Alkylierung, in an sich bekannter Weise in $X_1$: $-NH-A_1$ bzw. $-NH-A_2-X_2$ übergeführt werden.

BAD ORIGINAL

Zur Herstellung von Ausgangsverbindungen der Formel III, worin $X_4$ geschütztes Hydroxy bedeutet, kann man z.B. von einer Verbindung der Formel Va ausgehen, und die phenolische OH-Gruppe in an sich bekannter Weise schützen. Im folgenden Reaktionsschritt kann beispielsweise $Y_1$ in üblicher Weise zu Hydroxymethyl, Formyl bzw., ausgehend von verestertem Carboxy $Y_1$, zu verethertem Hydroxymethyl $R_1$ reduziert werden. Gewünschtenfalls wird Hydroxymethyl $R_1$ zu verestertem Hydroxymethyl $R_1$ verestert und Formyl $R_1$ zu acetalisiertem Formyl $R_1$ acetalisiert. Auf diese Weise resultieren entsprechenden Ausgangsverbindungen der Formel III, worin $X_4$ geschütztes Hydroxy bedeutet.

Ausgangsstoffe der Formel III, worin $X_4$ für eine Diazoniumgruppe mit einem Anion einer anorganischen oder organischen Säure als Gegenion steht, sind beispielsweise zugänglich, indem man von einer Verbindung der Formel

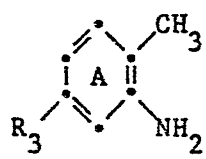 (Vm)

oder einem Salz davon ausgeht und die Aminogruppe gegebenenfalls durch Einführung einer Schutzgruppe schützt. Als Schutzgruppen kommen beispielsweise Acyl- oder Benzylgruppen in Betracht. Vorteilhafterweise wird die Aminogruppe, z.B. mit Benzylchlorid, benzyliert. Die sich hieran anschliessende Halogenierung der Methylgruppe, z.B. die Bromierung mit N-Bromsuccinimid in Gegenwart von Azobisisobutyronitril unter Erwärmen, führt zu den entsprechenden Verbindungen der Formel

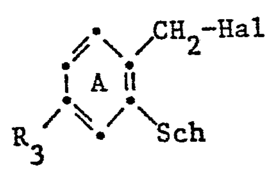 (Vn),

worin Hal Halogen, insbesondere Brom oder Chlor, bedeutet und Sch eine geschützte Aminogruppe darstellt. Diese Verbindungen werden anschliessend mit einem Alkalimetallcyanid, wie Natriumcyanid, beispielsweise unter Erwärmen in Dimethylformamid, umgesetzt. In den so erhältlichen Verbindungen der Formel

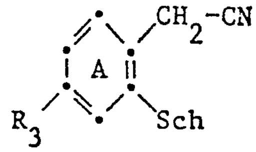

(Vo)

wird, sofern erwünscht, der Rest $R_2$ z.B. durch Umsetzung mit Verbindungen der Formel $R_2$-Hal (Vp) in Gegenwart einer Base, wie eines Alkalimetallhydrides, eingeführt. Im nächsten Reaktionsschritt wird die Cyanogruppe durch übliche Solvolyse in $Z_1$ überführt, $Z_1$ in geeigneter Weise in $R_1$ übergeführt, z.B. durch Reduktion mit einem Hydrid-übertragenden Reagenz, und anschliessend die Aminoschutzgruppe abgespalten. Vorteilhaft werden beispielsweise die die Aminogruppen schützenden Benzylgruppen hydrogenolytisch in Gegenwart eines Hydrierungskatalysators, z.B. Palladium, abgespalten.

Die so erhältlichen Verbindungen der Formel

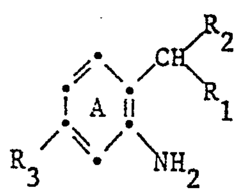

(Vq)

oder deren Salze werden z.B. bei niedrigen Temperaturen mit einer Mineralsäure, wie Schwefelsäure, und wässriger Alkalimetall-, wie Natriumnitritlösung, versetzt. Die dabei intermediär entstehenden Verbindungen der Formel (III), worin $X_4$ eine Diazoniumgruppe mit einem entsprechenden Gegenion bedeutet, kann wie vorstehend beschrieben, weiter zu Verbindungen der Formel (I) umgesetzt werden.

Die Verbindungen der Formeln Va und IV' entsprechen solchen Ausgangsverbindungen der Formel IV, worin $X_5$ für die Gruppe der Formel $-CH(R_2)-Y_1$ und $Y_1$ für gegebenenfalls funktionell abgewandeltes Carboxy stehen, und den entsprechenden Lactonen.
Verbindungen der Formel IV, worin $X_5$ für eine Gruppe der Formel

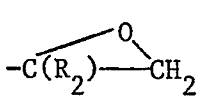

steht, sind beispielsweise zugänglich, indem man eine Verbindung der Formel Vc mit einem Gemisch von Trimethylsulfoniummethylsulfat und Natriummethylat umsetzt.

Die Bildung einer Gruppe der Formel $X_5 = -C(R_2)=CH-Y_4$, wobei $Y_4$ Wasserstoff ist, zur Herstellung von entsprechende Verbindungen der Formel IV, kann beispielsweise durch Umsetzung einer Verbindung der Formel Vc mit einem Grignard-Reagenz der Formel $Hal-Mg-CH_2-Y_4$ und sich anschliessender, z.B. säurekatalysierter Dehydratisierung erfolgen.

Ausgangsmaterial der Formel IV, worin $X_5$ für eine Gruppe der Formel $-C(R_2)=CH-Y_4$ steht und $Y_4$ verethertes Hydroxy darstellt, kann man beispielsweise herstellen, indem man eine Verbindung der Formel Vc mit einem entsprechenden Wittig-Reagenz, z.B. einer Verbindung der Formel $(C_6H_5)_3P=CH-Y_4$, umsetzt.

Eine erfindungsgemäss erhältliche Verbindung kann in an sich bekannter Weise in eine andere erfindungsgemässe Verbindung umgewandelt werden.

Falls der Ring A durch Niederalkylthio substituiert ist, kann man dieses auf übliche Weise zu entsprechendem Niederalkansulfinyl bzw. -sulfonyl oxidieren. Als geeignetes Oxidationsmittel für die Oxidation zur Sulfoxidstufe kommen beispielsweise anorganische Persäuren, wie Persäuren von Mineralsäuren, z.B. Periodsäure oder Perschwefelsäure, organische Persäuren, wie entsprechende Percarbon- oder Persulfonsäuren, z.B. Perameisen-, Peressig-, Trifluorperessig- bzw. Perbenzoesäure oder p-Toluolpersulfonsäure, oder Gemische aus Wasserstoffperoxid und Säuren, z.B. Gemisch aus Wasserstoffperoxid mit Essigsäure, in Betracht.

Häufig führt man die Oxidation in Gegenwart von geeigneten Katalysatoren durch, wobei als Katalysatoren geeignete Säuren, wie gegebenenfalls substituierte Carbonsäuren, z.B. Essigsäure oder Trifluoressigsäure, oder Uebergangsmetalloxide, wie Oxide von Elementen V. oder VI. Nebengruppe, z.B. Vanadium-, Molybdän- oder Wolframoxid, zu nennen sind. Die Oxidation wird unter milden Bedingungen, z.B. bei Temperaturen von etwa -50° bis etwa +100°C, durchgeführt.

Die Oxidation zur Sulfonstufe kann man auch mit Distickstofftetroxid als Katalysator in Gegenwart von Sauerstoff bei tiefen Temperaturen entsprechend durchführen, ebenso wie die direkte Oxidation des Niederalkylthio zum Niederalkansulfonyl. Jedoch setzt man hierbei üblicherweise das Oxidationsmittel im Ueberschuss ein.

Ist der Ring A durch Niederalkansulfinyl bzw. -sulfonyl substituiert, lässt sich dieser nach an sich bekannten Methoden zu entsprechendem Niederalkylthio, von Niederalkansulfonylderivaten ausgehend, auch zu Niederalkan-sulfinyl reduzieren. Als Reduktionsmittel eignen sich beispielsweise katalytisch aktivierter Wasserstoff, wobei Edelmetalle bzw. Oxide, wie Palladium, Platin oder Rhodium bzw. deren Oxide, verwendet werden, gegebenenfalls auf geeignetem Trägermaterial, wie Aktivkohle oder Bariumsulfat, aufgezogen. Weiterhin kommen reduzierende Metallkationen, wie Zinn II-, Blei II-, Kupfer I-, Mangan II-, Titan II-, Vanadium II-, Molybdän III- oder Wolfram III-Verbindungen, Halogenwasserstoffe, wie Chlor-, Brom- oder Iodwasserstoff, Hydride, wie komplexe Metallhydride, z.B. Lithiumaluminium-, Natriumbor-, Tributylzinnhydrid, Phosphorverbindungen, wie Phorphorhalogenide, z.B. Phosphortrichlorid, -bromid, Phosphorpentachlorid oder Phosphoroxichlorid, Phosphine, wie Triphenylphosphin, oder Phosphorpentasulfid-Pyridin, oder Schwefelverbindungen, wie Mercaptane, Thiosäuren, wie Thiophosphorsäuren oder Dithiocarbonsäuren, Dithionit oder Schwefel-Sauerstoff-Komplexe, wie Iod-Pyridin-Schwefeldioxidkomplex, in Frage.

Weist der aromatische Ring als Substituenten ein Wasserstoffatom auf, so kann dieses mit Hilfe eines Halogenierungsmittels in üblicher Weise durch ein Halogenatom ersetzt werden.

So erfolgt die Substitution von Wasserstoff durch Brom z.B. durch Bromierung mit Brom analog "Methoden der Organischen Chemie", Houben-Weyl (4. Edition), Vol, 5/4, S. 233-249, in einem inerten Lösungsmittel. Weiterhin kann mit Hilfe folgender Bromierungsmittel

bromiert werden: Hypobromsäure, Acylhypobromite oder andere organische Bromverbindungen, z.B. N-Bromsuccinimid, N-Bromacetamid, N-Bromphthalimid, Pyridiniumperbromid, Dioxandibromid, 1,3-Dibrom-5,5-dimethylhydantoin, 2,4,4,6-Tetrabrom-2,5-cyclohexadien-1-on.

Die entsprechende Chlorierung kann z.B. wie in Houben-Weyl (4. Edition), Vol. 5/3, S. 651-673, beschrieben durchgeführt werden, vorteilhaft mit elementaren Chlor, z.B. in einem halogenierten Kohlenwasserstoff, wie Chloroform, und unter Kühlen, z.B. auf etwa -10° bis etwa +10°C.

Der Ersatz von Wasserstoff durch Iod kann z.B. mit elementarem Iod in Gegenwart von Quecksilberoxid oder Salpetersäure erfolgen. Anstelle von elementem Iod lässt sich beispielsweise ein Alkalimetalliodid in Gegenwart eines Thallium(III)difluoracetat gemäss Tetrahedron Letters (1969), S. 2427 als Iodierungsmittel verwenden.

Ferner kann der Benzoteil des Ringsystems und/oder ein weiterer aromatischer Ring beispielsweise mit einem Niederalkanol bzw. einem Niederalkylhalogenid oder einem Phosphorsäureniederalkylester in Gegenwart von Lewis-Säuren alkyliert werden (Friedel-Crafts-Alkylierung). In einer Verbindung der Formel (I), worin ein aromatischer Ring Brom enthält, kann beispielsweise das Brom durch Umsetzung mit einem Niederalkylbromid in Gegenwart eines Alkalimetalls durch Niederalkyl ersetzt werden.

Enthält ein aromatischer Ring als Substituenten ein Wasserstoffatom, so kann dieses in an sich bekannter Weise durch eine Acylgruppe ausgetauscht werden. So kann beispielsweise die Einfügung der Acylgruppe analog der Friedel-Crafts-Acylierung (cf. G.A. Olah, Friedel-Crafts and Related Reactions, Vol. I, Interscience, New York, 1963-1965) durchgeführt werden, z.B. durch Umsetzung eines reaktiven funktionellen Acylderivates, wie eines Halogenids oder Anhydrids, einer organischen Carbonsäure in Gegenwart einer Lewis-Säure, wie Aluminium-, Antimon-(III)- oder -(V)-, Eisen(III)-, Zink(II)-chlorid oder Brotrifluorid.

Weisen Verbindungen der Formel I als Substituenten Hydroxy, so lässt sich dieses nach an sich bekannter Weise verethern. Die Umsetzung mit einer Alkoholkomponente, z.B. mit einem Niederalkanol, wie Ethanol, in Gegenwart von Säuren, z.B. Mineralsäure, wie Schwefelsäure, oder von Dehydratisierungsmitteln, wie Dicyclohexylcarbodiimid, führt zu Niederalkoxy. Umgekehrt kann man Ether spalten, indem man die Etherspaltung mittels Säuren, wie Mineralsäuren, z.B. Halogenwasserstoffsäure, wie Bromwasserstoffsäure, oder wie Lewissäuren, z.B. Halogeniden von Elementen der 3. Hauptgruppe, wie Bortribromid, oder mittels Pyridin-hydrochlorid oder Thiophenol durchführt.

Weiter lässt sich Hydroxy in Niederalkanoyloxy umwandeln, beispielsweise durch Umsetzung mit einer gewünschten Niederalkancarbonsäure, wie Essigsäure, oder einem reaktionsfähigen Derivat davon, beispielsweise in Gegenwart einer Säure, wie eine Protonsäure, z.B. Chlor-, Bromwasserstoff-, Schwefel-, Phosphor- oder einer Benzolsulfonsäure, in Gegenwart einer Lewissäure, z.B. von Bortrifluorid-Etherat, oder in Gegenwart eines wasserbindenden Mittels, wie Dicyclohexylcarbodiimid. Umgekehrt kann verestertes Hydroxy, z.B. durch Basen-Katalyse, zu Hydroxy solvolysiert werden.

Bedeutet $R_1$ Hydroxymethyl, kann dieses selektiv zu Formyl $R_1$ oxidiert werden. Als Oxidationsmittel verwendet man beispielsweise eine geeignete Chrom- oder Manganverbindung, wie ein Alkalimetall(di)chromat, ein Niederalkyl-, z.B. tert.-Butylchromat, das Jones-Reagens, ein Pyridin-Chromtrioxid-Komplex, Pyridinium-chlorchromat oder Mangandioxid. Weitere Oxidationssysteme sind beispielsweise der Dimethylsulfoxid-, z.B. Dicyclohexylcarbodiimid-Komplexe, z.B. in Gegenwart einer Protonquelle, Bleitetraacetat, Nickelperoxid, Silber(I)- und Kupfer(II)-Verbindungen, molekularer Sauerstoff in Gegenwart eines Hydrierungskatalysators, z.B. Platin, oder Aluminiumalkoxide, z.B. Aluminium-tert.-butylat und einem Ueberschuss an Keton, analog der Oppenhauer-Oxidation.

BAD ORIGINAL

Die Oxidation wird gegebenenfalls unter Kühlen oder Erwärmen durchgeführt, wobei es in einigen Fällen zweckmässig ist, die gebildete Formylverbindung aus dem Reaktionsmedium, z.B. durch Destillation, zu entfernen, um eine weitere Oxidation zu vermeiden.

Umgekehrt kann in einer erfindungsgemässen Verbindung, worin $R_1$ für Formyl steht, dieses durch Reduktion in Hydroxymethyl $R_1$ übergeführt, wobei als Reduktionsmittel z.B. elementarer Wasserstoff in Gegenwart eines Hydrierungskatalysators, z.B. Palladium auf Kohle, geeignete Hydrid-übertragende Reagentien, wie Alkalimetallaluminiumhydride, z.B. Lithiumaluminiumhydrid, Alkalimetallborhydride, z.B. Natriumborhydrid, oder Aluminiumalkanolate, z.B. Aluminiumisopropylat in Isopropanol, analog an Meerwein-Pondorff-Verley-Reduktion, verwendet werden können.

Bedeutet $R_1$ Formyl, kann dieses an sich bekannter Weise, z.B. durch säurekatalysiertes Behandeln mit einem Alkohol, z.B. Niederalkanol bzw. -diol, acetalisiert werden. Zweckmässig wird das bei der Reaktion gebildete Wasser, z.B. durch azeotrope Destillation oder Wasserbindende Mittel, wie Molekularsieb oder Natriumsulfat, entfernt. Umgekehrt kann acetalisiertes Formyl $R_1$ z.B. durch Behandeln mit wässriger Säure in Formyl $R_1$ übergeführt werden.

Wasserstoff $R_1$ kann beispielsweise durch Alkylierung, z.B. mit einem reaktionsfähigen veresterten Alkohol der Formel $R_1$-OH, z.B. einem entsprechenden Halogenid oder einem Sulfonyloxy-Derivat, insbesondere in Gegenwart einer starken Base, in Alkyl $R_1$ übergeführt werden. Die Alkylierung kann auch z.B. durch eine vorgelagerte Substitution von Wasserstoff $R_1$ durch Halogen und anschliessende Umsetzung mit Metallverbindungen der Formel $R_1$-Me erfolgen, wobei Me z.B. für ein Alkalimetall, Erdalkalimetallhalogenid oder Li-Cu steht.

Enthalten die Verbindungen der Formel (I) ungesättige Reste, wie Niederalkenyl oder Niederalkenylengruppierungen, können diese in an sich bekannter Weise in gesättigte Reste überführt werden. So erfolgt beispielsweise die Hydrierung von Mehrfachbindungen durch katalytische Hydrierung in Gegenwart von Hydrierungskatalysatoren, wobei hierfür z.B. Edelmetalle bzw. deren Derivate, z.B. Oxide, geeignet, wie Nickel, Raney-Nickel, Palladium, Platinoxid, die gegebenenfalls auf Trägermaterialien, z.B. auf Kohle oder Calciumcarbonat, aufgezogen sein können. Die Hydrierung kann vorzugsweise bei Drucken zwischen 1 und etwa 100 at. und bei Temperaturen zwischen etwa -80° bis etwa 200°C, vor allem zwischen Raumtemperatur und etwa 100°C durchgeführt werden. Die Reaktion erfolgt zweckmässig in einem Lösungsmittel, wie Wasser, einem Niederalkanol, z.B. Ethanol, Isopropanol oder n-Butanol, einem Ether, z.B. Dioxan, oder einer Niederalkancarbonsäure, z.B. Essigsäure.

Umgekehrt können in cyclischen Systemen $R_3$ eine oder mehrere Mehrfachbindungen eingeführt werden, Hierzu verwendet man geeignete Dehydrierungsmittel, beispielsweise Nebengruppenelemente, vorzugsweise solche der VIII Nebengruppe des Periodensystems, z.B. Palladium oder Platin, oder entsprechende Edelmetallderivate, z.B. Ruthenium-triphenylphosphid-chlorid, wobei die Mittel auf geeigneten Trägermaterialien aufgezogen sein können. Weitere bevorzugte Dehydrierungsmittel sind beispielsweise Chinone, wie p-Benzochinone, z.B. Tetrachlor-p-benzochinon oder 2,3-Dichlor-5,6-dicyano-p-benzochinon, oder wie Antrachinone, z.B. Phenanthren-9,10-chinon. Ferner können N-Halogensuccinimide, wie N-Chlorsuccinimid, Manganverbindungen, wie Bariummanganat oder Mangandioxid, und Selenderivate, wie Selendioxid oder Diphenylseleniumbis-trifluoracetat, verwendet werden.

Salze von Verbindungen der Formel (I) können in an sich bekannter Weise hergestellt werden. So erhält man beispielsweise Säureadditionssalze von Verbindungen der Formel (I) durch Behandeln mit einer Säure oder einem geeigneten Ionenaustauscherreagenz. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen mit salzbildenden, insbesondere basischen Eigenschaften in freier Form oder in Form von Salzen erhalten werden.

Infolge der engen Beziehung zwischen der neuen Verbindung in freier Form und in Form ihrer Salze sind im vorausgegangenen und nachfolgend unter der freien Verbindung oder ihren Salzen sinn- und zweckgemäss gegebenenfalls auch die entsprechenden Salze bzw. die freie Verbindung zu verstehen.

Die neue Verbindung einschliesslich ihrer Salze kann auch in Form ihrer Hydrate erhalten werden oder andere zur Kristallisation verwendete Lösungsmittel einschliessen.

Die neuen Verbindungen können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, oder als Salze vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch Chromatographie und/oder fraktionierte Kristallisation. Erhaltene Racemate lassen sich ferner nach bekannten Methoden in die optischen Antipoden zerlegen, beispielsweise durch Um-

BAD ORIGINAL

kristallisation aus einem optisch aktiven Lösungsmittel, mit Hilfe von Mikroorganismen oder durch Ueberführung in diastereomere Salze oder Ester, z.B. durch Umsetzung eines sauren Endstoffes mit einer mit der racemischen Säure Salze bildenden optisch aktiven Base oder einer optisch aktiven Carbonsäure oder einem reaktiven Derivat davon, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen führen. Neue Ausgangsstoffe, die speziell für die Herstellung der erfindungsgemässen Verbindungen entwickelt werden, ihre Verwendung, z.B. als Arzneimittelwirkstoffe, Formulierungsverfahren und Verfahren zu ihrer Herstellung bilden ebenfalls einen Gegenstand der Erfindung, $R_1$, $R_2$, $R_3$ und A die für die jeweils bevorzugten Verbindungsgruppen der Formel I angegebenen Bedeutungen haben.

Bei den erfindungsgemässen pharmazeutischen Präparaten, welche die erfindungsgemässe Verbindung oder pharmazeutisch verwendbare Salze davon enthalten, handelt es sich um solche zur topischen Anwendung, zur enteralen, wie oralen oder rektalen, und parenteralen Verabreichung an Warmblüter(n), wobei der pharmakologische Wirkstoff allein oder zusammen mit einem pharmazeutisch anwendbaren Trägermaterial enthalten ist. Die tägliche Dosierung des Wirkstoffs

BAD ORIGINAL

hängt von dem Alter und dem individuellen Zustand, sowie von der Applikationsweise ab.

Die neuen pharmazeutischen Präparate enthalten z.B. von etwa 10 % bis etwa 80 %, vorzugsweise von etwa 20 % bis etwa 60 % des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliess-, Regulier- und Schmiermittel, z.B. Kieselsäure, Talk, Stearinsäure oder Salze davon, wie Magnesium- oder Calciumstearat,

BAD ORIGINAL

und/oder Polyethylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyethylenglykolen, gelöst oder supsendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositorienmasse eignen sich z.B. natürliche oder synthetische Triglyceride, Paraffinkohlenwasserstoffe, Polyethylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyethylenglykole oder Paraffinkohlenwasserstoffe in Frage.

BAD ORIGINAL

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Ethyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Als topisch anwendbare pharmazeutische Präparate kommen in erster Linie Creme, Salben, Pasten, Schäume, Tinkturen und Lösungen in Frage, die von etwa 0,1 bis etwa 5 % des Wirkstoffs enthalten.

Creme sind Oel-in-Wasser Emulsionen die mehr als 50 % Wasser aufweisen. Als ölige Grundlage verwendet man in erster Linie Fettalkohole, z.B. Lauryl-, Cetyl- oder Stearylalkohol, Fettsäuren, z.B. Palmitin- oder Stearinsäure, flüssige bis feste Wachse, z.B. Isopropylmyristat, Wollwachse oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitanfettsäureester (Tweens), ferner Polyoxyethylenfettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Creme verhindern, z.B. Polyalkohole, wie Glycerin, Sorbit, Propylenglykol und/oder Polyethylenglykole, ferner Konservierungsmittel, Riechstoffe etc.

BAD ORIGINAL

Salben sind Wasser-in-Oel-Emulsionen, die bis zu 70 %, vorzugsweise jedoch von etwa 20 % bis etwa 50 % Wasser oder wässrige Phasen enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole bzw. Wollwachse enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind u.a. Feuchthaltungsmittel, wie Polyalkohole, z.B. Glycerin, Propylenglykol, Sorbit und/oder Polyethylenglykol, sowie Konservierungsmittel, Riechstoffe, etc.

Fettsalben sind wasserfrei und enthalten als Grundlage insbesondere Kohlenwasserstoff, z.B. Paraffin, Vaseline und/oder flüssige Paraffine, ferner natürliches oder partialsynthetisches Fett, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasseraufnahmefähigkeit steigernden Fettalkohole, Emulgatoren und/oder Zusätze.

Pasten sind Creme und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden z.B. aus Druckbehältern verabreicht und sind in Aerosolform vorliegende flüssige Oel-in Wasser-Emulsionen, wobei halogenierte Kohlenwasserstoffe, wie Chlorfluorniederalkane, z.B. Dichloridfluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässerig-ethanolische Grundlage auf, der u.a. Polyalkohole, z.B. Glycerin, Glykole, und/oder Polyethylenglykol, Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Ethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei der Verarbeitung des Wirkstoffs als Lösung wird dieser in der Regel von der Emulgierung in einer der beiden Phasen gelöst; bei Verarbeitung als Suspension wird er nach der Emulgierung mit einem Teil der Grundlage vermischt und dann dem Rest der Formulierung beigegeben.

Die Dosierung des Wirkstoffs hängt von der Warmblüter-Spezies dem Alter und dem individuellen Zustand sowie der Applikationsweise ab. Im Normalfall ist für einen etwa 75 kg schweren Warmblüter bei oraler Applikation eine ungefähre Tagesdosis von etwa 100 bis etwa 600 mg vorteilhaft in mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

BAD ORIGINAL

Beispiel 1:

Zu einer Suspension von 0,8 g Lithiumaluminiumhydrid (0,02 Mol) in 50 ml abs. Ether werden unter Stickstoffatmosphäre, Rühren und Eiskühlung bei 0-5° innert 30 Minuten 2,7 g (0,01 Mol) 5-Chlor-3-methyl-6-morpholino-benzofuran-2(3H)-on gelöst in 100 ml abs. Ether getropft. Anschliessend wird 3 Stunden bei Raumtemperatur gerührt. Durch vorsichtiges Zutropfen von ca. 10 ml Wasser unter Eiskühlung wird der Lithiumaluminiumkomplex zerlegt. Mittels 1n Salzsäure wird schwach angesäuert und 5 mal mit Chloroform extrahiert. Das so erhaltene Rohprodukt wird aus Essigester umkristallisiert. Auf diese Weise wird 2-(5-Chlor-2-hydroxy-4-morpholino-phenyl)-propan-1-ol vom Smp. 176-177° isoliert.

Das Ausgangsmaterial kann beispielsweise wie folgt erhalten werden: Ein Gemisch von 341 g (2 Mol) des Hydrochlorids von Imidazol[1,2-a]-pyridin-2-(3H)-on in 700 ml Wasser wird unter Rühren portionsweise mit einer heissen Lösung von 80 g (2 Mol) Natronlauge in 200 ml Wasser versetzt. Anschliessend wird eine Lösung von 250,7 g (2,16 Mol) Maleinsäure in 600 ml Wasser so zugetropft, dass die Innentemperatur des Reaktionsgemisches zwischen 40°C und 45°C bleibt. Nach 30 Stunden bei Raumtemperatur (20-25°C) wird auf 5°C gekühlt, der gebildete Niederschlag abfiltriert, das Filtrat im Vakuum auf etwa die Hälfte eingeengt und das ausgefallene Produkt abgenutscht. Die vereinigten Rückstände werden mit wenig kaltem Methanol gewaschen und bei 50°C im Vakuum getrocknet. Es resultieren 400 g 3-(1,2-Dicarboxyethyl)-imidazo[1,2-a]pyridin-2(3H)-on vom Smp. 193° (Zers.). Das erhaltene Produkt wird bei Raumtemperatur während 6 Stunden mit 650 ml konz. Salzsäure verrührt. Nach Kühlen auf 5°C wird der Niederschlag abfiltriert, das Filtrat im Vakuum auf etwa die Hälfte eingeengt und das ausgefallene Produkt abgenutscht. Die vereinigten Rückstände werden mit Aceton gewaschen und bei 50°C im Vakuum getrocknet. Man erhält so das Hydrochlorid von 3-(1,2-Dicarboxyethyl)-imidazo[1,2-a]pyridin-2(3H)-on vom Smp. 205°C (Zers.).

Ein Gemisch von 114,7 g (0,4 Mol) des Hydrochlorids von 3-(1,2-Dicarboxyethyl)-imidazo[1,2-a]pyridin-2(3H)-on, 36,4 g (0,52 Mol) Methyl-vinylketon, 150 ml Methanol und 150 ml Wasser wird während 36 Stunden bei Raumtemperatur gerührt und anschliessend bei ca. 45°C im Vakuum zur Trockene eingedampft. Das erhaltene Rohprodukt wird in 300 ml Eisessig aufgenommen, mit 15 g Natriumacetat versetzt und bis zur beendeten $CO_2$-Entwicklung am Rückfluss gekocht. Anschliessend wird das Lösungsmittel im Vakuum entfernt, der Rückstand mit einem Gemisch von 150 ml 6M Schwefelsäure und 150 ml Tetrahydrofuran versetzt und während 8 Stunden bei 60°C gehalten. Nach dem Entfernen des Tetrahydrofurans im Vakuum wird das Reaktionsgemisch mit Wasser verdünnt, mit Methylenchlorid ausgezogen und über Kieselgel filtriert. Destillation des Rohproduktes im Hochvakuum (115°C-125°C/8 Pa) ergibt das 4-Methyl-3-(3-oxo-butyl)-maleinsäureanhydrid als spektroskopisch einheitliches blassgelbes Oel.

Ein Gemisch von 18,2 g (0,1 Mol) 4-Methyl-3-(3-oxobutyl)-maleinsäureanhydrid und 22 g (0,105 Mol) Morpholiniumbenzoat in 400 ml Benzol wird während 48 Stunden am Wasserabscheider unter Rückfluss erhitzt. Das Benzol wird im Vakuum entfernt, der Rückstand in Methylenchlorid aufgenommen und die organische Phase zweimal mit gesättigter Natriumbicarbonatlösung extrahiert. Das nach dem Trocknen und Entfernen des Methylenchlorids verbleibende Rohprodukt wird mit Petrolether/Ether an Kieselgel chromatographiert. Es werden blassgelbe Kristalle erhalten, die aus Methylenchlorid/Ether umkristallisiert werden. Man erhält so das 3-Methyl-6-morpholino-benzofuran-2(3H)-on vom Smp. 118-121°C.

Zu einem Gemisch aus 14,7 g (0,063 Mol) 3-Methyl-6-morpholino-benzofuran-2(3H)-on in 100 ml Chloroform wird bei 0-5°C unter Rühren eine kalte Lösung von Chlor in Chloroform getropft, bis im Dünnschichtchromatogramm kein Edukt mehr sichtbar ist. Das Reaktionsgemisch wird mit Methylenchlorid verdünnt und nacheinander mit 10%iger

BAD ORIGINAL

Natriumthiosulfatlösung, verdünnter Natriumbicarbonatlösung und Wasser gewaschen. Das nach dem Trocknen und Eindampfen der organischen Phase verbleibende Rohprodukt wird mit Petrolether/Ether an Kieselgel chromatographiert. Nach Umkristallisation der reinen Fraktionen aus Ether/Petrolether erhält man das 5-Chlor-3-methyl-6-morpholino-benzofuran-2(3H)-on vom Smp. 103-105°C.

Beispiel 2:

11,35 g (0,05 Mol) 3,5-Dimethyl-6-(pyrrol-1-yl)-benzofuran-2(3H)-on werden in 250 ml Tetrahydrofuran gelöst und unter Rühren bei Raumtemperatur, innert einer Stunde, 1,9 g (0,05 Mol) Natriumborhydrid portionsweise eingetragen. Die Temperatur steigt dabei auf 36°. Nach weiteren 12 Stunden Rühren wird im Vakuum zur Trockne verdampft, der Rückstand zwischen Ether und Wasser verteilt und der Ether mit verdünnter Salzsäure und Wasser gewaschen. Nach dem Trocknen und Abdampfen des Ethers wird der Rückstand aus Isopropylether kristallisiert. Man erhält so 2-[2-Hydroxy-5-methyl-4-(pyrrol-1-yl)-phenyl]-propan-1-ol vom Smp. 117-118°.

Das Ausgangsmaterial kann wie folgt hergestellt werden:

Ein Gemisch aus 172 g(0,6 Mol) Hydrochlorid von 3-(1,2-Dicarboxyethyl)-imidazol[1,2-a]pyridin-2(3H)-on, 65,5 g (0,78 Mol) 3-Methyl-3-buten-2-on, 220 ml Methanol und 220 ml Wasser wird während 36 Stunden bei Raumtemperatur gerührt und anschliessend bei ca. 45°C im Vakuum zur Trockene eingedampft. Das erhaltene Rohprodukt wird in 400 ml Eisessig aufgenommen, mit 22,5 g Natriumacetat versetzt und bis zur beendeten $CO_2$-Entwicklung am Rückfluss gekocht. Anschliessend wird das Lösungsmittel im Vakuum entfernt, der Rückstand mit einem Gemisch aus 225 ml 6 M Schwefelsäure und 225 ml Tetrahydrofuran versetzt und während 8 Stunden am Rückfluss erhitzt. Nach dem Entfernen von Tetrahydrofuran im Vakuum wird das Reaktionsgemisch mit Wasser verdünnt und mit Methylenchlorid ausgezogen. Das nach dem Trocknen und Eindampfen der organischen Phase verbleibende Rohprodukt wird mit

BAD ORIGINAL

Petrolether/Ether an Kieselgel chromatographiert. Die anschliessende Destillation (100° C/8·$10^{-2}$ Torr) ergibt 4-Methyl-3-(2-methyl-3-oxobutyl)-maleinsäureanhydrid als blassgelbes Oel.

59 g 4-Methyl-3-(2-methyl-3-oxo-butyl)-maleinsäureanhydrid und 240 g Dibenzylammoniumbenzoat werden in 1000 ml Benzol mit einem Wasserabscheider 48 Stunden am Rückfluss gekocht. Anschliessend wird im Vakuum zur Trockene eingedampft und der Rückstand über Kieselgel chromatographiert. Das erhaltene Oel kristallisiert aus Isopropylether. Man erhält so das 2-(4-Dibenzylamino-2-hydroxy-5-methyl-phenyl)-propionsäure-dibenzylamid vom Smp. 140-141°C.

20 g 2-(4-Dibenzylamino-2-hydroxy-5-methyl-phenyl)-propionsäure-dibenzylamid werden in 40 ml 2n Salzsäure und 40 ml Eisessig 3 Stunden am Rückfluss erhitzt. Anschliessend wird im Vakuum zur Trockene eingedampft und der Rückstand zwischen Ether und 1n Natronlauge verteilt. Durch Ansäuern auf pH 1 mit Salzsäure und Extrahieren mit Ether erhält man die 2-[4-Dibenzylamino-2-hydroxy-5-methyl-phenyl)-propionsäure, die zur Reinigung in Methylenchlorid über Kieselgel chromatographiert wird. Die farblosen Kristalle schmelzen bei 174-175°C. 5 g davon werden in 50 ml Ether gelöst und mit 6 g Dicyclohexylcarbodiimid versetzt. Nach 30 Minuten wird vom gebildeten Harnstoff abfiltriert und das Filtrat zur Trockene eingedampft. Man erhält so das 6-Dibenzylamino-3,5-dimethyl-benzofuran-2(3H)-on vom Smp. 122-123°C.

4 g 6-Dibenzylamino-3,5-dimethyl-benzofuran-2(3H)-on werden in 40 ml Dioxan gelöst und mit 0,4 g Palladium auf Kohle (5%ig) bei Raumtemperatur unter Normaldruck mit Wasserstoff reduziert. Anschliessend wird filtriert, das Filtrat zur Trockene eingedampft und aus Methanol umkristallisiert. Man erhält so das 6-Amino-3,5-dimethyl-benzofuran-2(3H)-on vom Smp. 123-124°C.

BAD ORIGINAL

1,8 g 6-Amino-3,5-dimethyl-benzofuran-2(3H)-on werden in 20 ml Dioxan gelöst und unter Rühren bei Raumtemperatur mit 2 ml 2,5-Dimethoxy-tetrahydrofuran und 1,4 ml 37%iger Salzsäure versetzt. Nach 30 Minuten wird die wässrige Phase abgetrennt und die organische Phase im Vakuum zur Trockene eingedampft. Der Rückstand wird in Methylenchlorid über Kieselgel chromatographiert. Das erhaltene hellgelbe Oel kristallisiert aus Dibutyloxid. Man erhält so das 3,5-Dimethyl-6-(pyrrol-1-yl)-benzofuran-2(3H)-on vom Smp. 61-63°C.

Beispiel 3:

Zu einer Suspension von 1,20 g (30 mMol) Lithiumaluminiumhydrid in 100 ml abs. Ether werden unter Stickstoffatmosphäre, Rühren und Eiskühlung bei 0-5° innert 30 Minuten 2,45 g (10 mMol) 2-[2-Hydroxy-5-methyl-4-(pyrrol-1-yl)-phenyl]-propionsäure in 100 ml abs. Ether getropft. Anschliessend wird 5 Stunden bei Raumtemperatur gerührt. Der Lithiumaluminiumkomplex wird unter Eiskühlung durch Zutropfen von 12 ml Wasser zerlegt. Mittels verdünnter Salzsäure wird schwach angesäuert, die Etherphase abgetrennt und die wässrige Phase noch zweimal mit Ether extrahiert. Die Etherphasen werden mit Wasser gewaschen, vereinigt, über Natriumsulfat getrocknet und am Vakuumrotationsverdampfer eingeengt. Umkristallisation des Rohprodukts aus Diisopropylether liefert 2-[2-Hydroxy-5-methyl-4-(pyrrol-1-yl)-phenyl]-propanol vom Smp. 117-118°.

Das Ausgangsmaterial kann beispielsweise folgendermassen hergestellt werden:

Eine Lösung von 132,9 g (0,759 Mol) 4-Methyl-3-nitro-anisol in 1,1 Liter Methanol wird mit 12,4 g Palladium auf Kohle versetzt und bei Raumtemperatur hydriert. Der Katalysator wird abfiltriert und das Filtrat am Vakuumrotationsverdampfer eingeengt. Umkristallisation aus Isopropanol/Wasser ergibt 3-Amino-4-methyl-anisol vom Smp. 43-44°.

Eine Lösung von 88,4 g (0,64 Mol) 3-Amino-4-methyl-anisol in 1,4 Liter Eisessig wird auf 106° erwärmt und bei dieser Temperatur während 30 Minuten mit 114 g (0,86 Mol) 2,5-Dimethoxy-tetrahydrofuran versetzt. Anschliessend wird sofort auf Raumtemperatur abgekühlt und am Vakuumrotationsverdampfer eingeengt. Destillation des Rückstandes am Hochvakuum liefert das 4-Methyl-3-(pyrrol-1-yl)-anisol, Kp. 93-95°/0,04 Torr. $R_f$ (Toluol/Essigester =10:1):0, 57.

Eine Lösung von 86,6 g (0,46 Mol) 4-Methyl-3-(pyrrol-1-yl)-anisol in 1,5 Liter absolutem Methylenchlorid wird mit Aceton/Trockeneis auf -78° abgekühlt. Bei dieser Temperatur werden 231,7 g (0,92 Mol) Bortribromid zugetropft. Anschliessend wird das Kühlbad entfernt und das Reaktionsgemisch auf 0 bis 5° erwärmt, dann auf 2 Liter Eis /Wasser gegossen, die Methylenchloridphase abgetrennt und mit gesättigter Kochsalzlösung gewaschen. Die wässrigen Phasen werden noch 2 mal mit Methylenchlorid nachextrahiert. Die organischen Phasen werden vereinigt, über Natriumsulfat getrocknet und am Vakuumrotationsverdampfer eingeengt. Destillation des Rückstandes am Hochvakuum ergibt das 4-Methyl-3-(pyrrol-1-yl)-phenol, Kp. 105-107°/ 0,03 Torr, $R_f$ (Toluol/Essigester = 10:1):0,38.

Eine Suspension von 53,4 g (0,31 Mol) 4-Methyl-3-(pyrrol-1-yl)-phenol und 53,7 g (0,39 Mol) Kaliumcarbonat in 60 ml absolutem Aceton wird unter Rückfluss während 1 Stunde mit 45,7 g (0,39 Mol) Crotylbromid versetzt und anschliessend noch $4\frac{1}{2}$ Stunden weitergekocht. Das Reaktionsgemisch wird abgekühlt und mit 800 ml Wasser verdünnt. Das Aceton wird am Vakuumrotationsverdampfer abgedampft und der Rückstand mehrmals mit Methylenchlorid extrahiert. Die organischen Phasen werden mit Wasser gewaschen, vereinigt und über Natriumsulfat getrocknet und am Vakuumrotationsverdampfer eingeengt. Kurzfiltration an etwa 800 g Kieselgel mit Methylenchlorid ergibt 1-[4-Methyl-

3-(pyrrol-1-yl)]-phenoxy-2-buten als hellgelbes Oel, $R_f$ (Hexan/Ether = 9:1):0,45, $R_f$ (Toluol/Essigester = 10:1):0,68.

Eine Lösung von 60 g (0,26 Mol) 1-[4-Methyl-3-(pyrrol-1-yl)]-phenoxy-2-buten in 170 ml absolutem N,N-Diethylanilin wird während 5 Stunden unter Rückfluss gekocht. Das Reaktionsgemisch wird abgekühlt, mit Methylenchlorid verdünnt und mit 6N Salzsäure angesäuert. Die Wasserphase wird abgetrennt und nochmals mit Methylenchlorid extrahiert. Die organischen Phasen werden neutral gewaschen, vereinigt, über Natriumsulfat getrocknet und am Vakuumrotationsverdampfer eingeengt. Chromatographie an Kieselgel mit Hexan/Ether (9:1) ergibt 3-[2-Hydroxy-5-methyl-4-(pyrrol-1-yl)-phenyl]-1-buten. $R_f$ (Hexan/Ether = 9:1):0,17, $R_f$ (Toluol/Essigester = 10:1):0,45.

Eine Lösung von 26,7 g (0,12 Mol) 3-[2-Hydroxy-5-methyl-4-(pyrrol-1-yl)-phenyl]-1-buten in 370 ml Essigsäureanhydrid wird mit einigen Tropfen Pyridin versetzt und während 2 Stunden bei Raumtemperatur gerührt. Das Reaktionsgemisch wird auf Eis gegossen und mit Methylenchlorid 3 mal extrahiert.Die Methylenchloridphasen werden mit verdünnter Natriumbicarbontlösung, dann mit Wasser neutral gewaschen, vereinigt über Natriumsulfat getrocknet und am Vakuumrotationsverdampfer eingeengt. Filtration an wenig Kieselgel mit Methylenchlorid ergibt 3-[2-Acetoxy-5-methyl-4-(pyrrol-1-yl)-phenyl]-1-buten, $R_f$ (Toluol/Essigester = 10:1):0,55.

Eine Lösung von 2,7 g (10 mMol) 3-[2-Acetoxy-5-methyl-4-(pyrrol-1-yl)-phenyl]-1-buten in 40 ml absolutem Methylenchlorid wird mit Aceton /Trockeneis auf -78° abgekühlt und solange Ozon durchgeblasen bis die blaue Farbe nicht mehr verschwindet. Dann werden 2 ml Dimethylsulfid zugegeben und das Kühlbad entfernt. Das Reaktionsgemisch wird am Vakuumrotationsverdampfer schonend eingeengt, der Rückstand in 50 ml Ethanol gelöst und mit einer Lösung von 3,7 g (23 mMol)

Silbernitrat in 5 ml Wasser versetzt. Zu diesem Gemisch wird während etwa 15 Minuten eine Lösung von 75 ml einer 1N Kaliumhydroxidlösung getropft. Das heterogene Gemisch wird während weiteren 2 Stunden weitergerührt. Das Reaktionsgemisch wird filtriert und der Rückstand mit Ethanol gewaschen. Das alkalische Filtrat wird über Nacht bei Raumtemperatur stehengelassen und mit Methylenchlorid extrahiert. Die alkalische Lösung wird mit 6N Salzsäure unter Kühlung vorsichtig angesäuert und mit Methylenchlorid mehrmals extrahiert. Die organischen Phasen werden noch zweimal mit Wasser gewaschen, vereinigt, über Natriumsulfat getrocknet und am Vakuumrotationsverdampfer eingeengt. Umkristallisation aus Diisopropylether/Petrolether ergibt 2-[2-Hydroxy-5-methyl-4-(pyrrol-1-yl)-phenyl]-propionsäure vom Smp. 73-74°.

Das Ausgangsmaterial kann beispielsweise ebenfalls wie folgt hergestellt werden:

Eine Lösung von 2,5 g (10 mMol) 2-[2-Hydroxy-5-methyl-4-(pyrrol-1-yl)-phenyl]-propionsäure in 30 ml absolutem Methylenchlorid wird bei Raumtemperatur mit 2,27 g (11 mMol) Dicyclohexylcarbodiimid in 20 ml absolutem Methylenchlorid versetzt. Das Reaktionsgemisch wird ca. 30 Minuten bei Raumtemperatur gerührt, der Niederschlag abfiltriert und das Filtrat am Vakuumrotationsverdampfer eingeengt. Destillation ergibt 3,5-Dimethyl-6-(pyrrol-1-yl)-benzofuran-2(3H)-on, Kp. 180°/0,005 Torr, Fp. 61-63°.

Beispiel 4:

Eine Lösung von 3,57 g (10,0 mMol) 2-[2-Benzyloxy-5-chlor-4-(piperidin-1-yl)phenyl]-propionaldehyd in 40 ml Dioxan wird mit 0,5 g 10 proz. Palladium-Kohle versetzt und während 20 Stunden bei Raumtemperatur hydriert. Anschliessend wird vom Katalysator abfiltriert und im Vakuum eingedampft. Destillation (120-130°/0,02 Torr) ergibt 2-[5-Chlor-2-hydroxy-4-(piperidin-1-yl)phenyl]-propanol.

BAD ORIGINAL

Das Ausgangsmaterial kann beispielsweise wie folgt hergestellt werden:

Zu einer Suspension von 101,5 g (0,40 Mol) 5-Chlor-2-hydroxy-4-(piperidin-1-yl)-acetophenon, 60,8 g (0,44 Mol) gemahlenem Kaliumcarbonat, 39,9 g (0,267 Mol) Natriumiodid und 900 ml Dimethylformamid werden bei Raumtemperatur unter Rühren während 30 Minuten 55,7 g (0,44 Mol) Benzylchlorid getropft. Anschliessend wird drei Stunden bei 50° gehalten, abgekühlt, genutscht und der Rückstand mit Dimethylformamid nachgewaschen. Die organische Lösung wird am Vakuumrotationsverdampfer eingeengt, der Rückstand in Methylenchlorid aufgenommen und mit Wasser gewaschen. Der nach dem Trocknen und Entfernen des Methylenchlorids verbleibende Rückstand wird mit Petrolether/Methylenchlorid an Kieselgel chromatographiert. Nachfolgende Destillation (Sdp. 150-160°/0,01 Torr) ergibt 2-Benzyloxy-5-chlor-4-(piperidin-1-yl)-acetophenon.

Ein Gemisch von 171,9 g (0,50 Mol) 2-Benzyloxy-5-chlor-4-(piperidin--1-yl)-acetophenon, 54,5 ml (0,50 Mol) Chloressigsäureethylester und 100 ml abs. Benzol wird während zwei Stunden unter Rühren mit 23,6 g (0,60 Mol) fein pulverisiertem Natriumamid versetzt, wobei die Innentamperatur zwischen 15 und 20° gehalten wird. Nach weiterem Rühren während zwei Stunden wird das Reaktionsgemisch auf 350 g Eis gegossen. Die organische Phase wird abgetrennt und die wässrige Phase noch zweimal mit Benzol extrahiert. Die organischen Extrakte werden mit Wasser gewaschen, vereinigt, über Natriumsulfat getrocknet und am Vakuumrotationsverdampfer eingeengt. Kurzwegdestillation bei 180°-190°/0,01 Torr ergibt 1-[2-Benzyloxy-5-chlor-4-(piperidin-1-yl)-phenyl]-2-ethoxycarbonyl-1-methyl-ethylenoxid.

Eine Lösung von 215,0 g (0,50 Mol) 1-[2-Benzyloxy-5-chlor-4-(piperidin-1-yl)phenyl]-2-ethoxycarbonyl-1-methyl-ethylenoxid in 1,5 l Ethanol wird mit 250 ml einer 4N Natriumhydroxid-Lösung versetzt und über Nacht bei Raumtemperatur stehen gelassen. Nach dem Ansäuern mit eisgekühlter verdünnter Salzsäure wird das Ethanol am Vakuumrotationsverdampfer schonend abdestilliert. Der Rückstand wird mit Wasser verdünnt und dreimal mit Methylenchlorid extrahiert. Die organischen Extrakte werden mit Wasser gewaschen, vereinigt, über Natriumsulfat getrocknet und am Vakuumrotationsverdampfer eingeengt. Das relativ instabile 1-[2-Benzyloxy-5-chlor-4-(piperidin-1-yl)-phenyl]-2-carboxy-1-methyl-ethylenoxid wird ohne Reinigung für die weiteren Umsetzungen verwendet.

120,6 g (0,30 Mol) des rohen 1-[2-Benzyloxy-5-chlor-4-(piperidin-1-yl)-phenyl]-2-carboxy-1-methyl-ethylenoxid werden mit 1,1 g Kupferpulver versetzt und am Wasserstrahlvakuum bis zur beendeten Decarboxylierung bei einer Oelbadtemperatur von 130° gehalten. Der so erhaltene 2-[2-Benzyloxy-5-chlor-4-(piperidin-1-yl)phenyl]-propionaldehyd wird bei 150-170°/0,01 Torr destilliert.

Beispiel 5:

Eine Lösung von 22,9 g (0,074 Mol) 1-[2-Acetoxy-5-chlor-4-(piperidin-1-yl)phenyl]-1-methyl-ethylenoxid in 135 ml abs. Benzol wird mit 6,4 g (45 mMol) frisch destilliertem Bortrifluoridetherat versetzt und während 5 Minuten heftig gerührt. Das Reaktionsgemisch wird mit Wasser versetzt. Die Benzolphase wird abgetrennt, mit Wasser gewaschen, über Natriumsulfat getrocknet und am Vakuumrotationsverdampfer eingeengt. Destillation (Sdp. 105-115°/0,02 Torr) ergibt 2-[2-Acetoxy-5-chlor-4-(piperidin-1-yl)phenyl]-propionaldehyd.

Das Ausgangsmaterial kann beispielsweise wie folgt hergestellt werden:

Ein Gemisch von 2,54 g (10,0 mMol) 5-Chlor-2-hydroxy-4-(piperidin-1-yl)-acetophenon und 30 ml Essigsäureanhydrid wird während 3 Stunden unter Rückfluss gekocht. Das Reaktionsgemisch wird am Vakuumrotationsverdampfer eingeengt und der Rückstand zwischen Methylenchlorid und verdünnter Bicarbonat-Lösung verteilt. Das nach dem Trocknen und Einengen der organischen Phase erhaltene Rohprodukt wird mit Petrolether/

Methylenchlorid an Kieselgel chromatographiert. Anschliessende Destillation (Sdp. 100-110°/0,01 Torr) ergibt 2-Acetoxy-5-chlor-4-(piperidin-1-yl)-acetophenon.

Zu einer Lösung von 18,9 g (0,15 Mol) Dimethylsulfat in 70 ml Acetonitril wird bei Raumtemperatur eine Lösung von 10,3 g (0,165 Mol) Dimethylsulfid in 30 ml Acetonitril gegeben. Es wird über Nacht stehen gelassen, dann bei Raumtemperatur mit 8,9 g (0,165 Mol) Natriummethylat versetzt. Anschliessend werden während 15 Minuten 29,5 g (0,10 Mol) 2-Acetoxy-5-chlor-4-(piperidin-1-yl)-acetophenon zugegeben. Dimethylsulfid und Lösungsmittel werden grösstenteils abdestilliert, der Rückstand mit Wasser versetzt und mit Methylenchlorid extrahiert. Die organischen Phasen werden mit Wasser gewaschen, vereinigt, über Natriumsulfat getrocknet und am Vakuumrotationsverdampfer eingeengt. Destillation des Rückstandes (Sdp. 105-115°/0,02 Torr) ergibt 1-[2-Acetoxy-5-chlor-4-(piperidin-1-yl)phenyl]-1-methyl-ethylenoxid.

Beispiel 6:

Eine Lösung von 3,1 g (10,0 Mol) 2-[2-Acetoxy-5-chlor-4-(piperidin-1--yl)phenyl]-propionaldehyd in 50 ml Tetrahydrofuran wird mit 0,6 g 10 proz. Palladium-Kohle versetzt und während 15 Stunden bei Raumtemperatur hydriert. Anschliessend wird vom Katalysator abfiltriert und im Vakuum eingedampft. Destillation (Sdp. 110-120°/0,01 Torr) ergibt 2-[2-Acetoxy-5-chlor-4-(piperidin-1-yl)phenyl]-propanol.

Beispiel 7:

Ein Gemisch von 0,1 g Ammoniumnitrat und 6,2 g (20 mMol) 2-[2-Acetoxy-5-chlor-4-(piperidin-1-yl)phenyl]-propionaldehyd in 10 ml abs. Ethanol wird mit 6,7 ml (40 mMol) Ortoameisensäure-triethylester versetzt und während 6 Stunden bei Raumtemperatur gerührt. Das Reaktionsgemisch wird anschliessend mit Eiswasser hydrolisiert und dreimal mit

Methylenchlorid extrahiert. Die organischen Phasen werden mit Wasser gewaschen, vereinigt, über Natriumsulfat getrocknet und am Vakuumrotationsverdampfer eingeengt. Destillation des Rückstands (Sdp. 170-180°/0,04 Torr) ergibt 2-[2-Acetoxy-5-chlor-4-(piperidin-1-yl)phenyl]-propionaldehyddiethylacetal.

Beispiel 8:

Eine Lösung von 3,83 g (10,0 mMol) 2-[2-Acetoxy-5-chlor-4-(piperidin--1-yl)phenyl]-propionaldehyddiethylacetal in 30 ml Ethanol wird mit 10 ml einer 2N Natronlauge-Lösung versetzt und während 5 Stunden bei Raumtemperatur gerührt. Mit verdünnter Salzsäure-Lösung wird unter Eiskühlung neutralisiert und dreimal mit Methylenchlorid extrahiert. Die organischen Phasen werden mit Wasser gewaschen, vereinigt, über Natriumsulfat getrocknet und am Vakuumrotationsverdampfer eingeengt. Chromatographie des Rückstandes mit Petrolether/Methylenchlorid an Kieselgel ergibt 2-[5-Chlor-2-hydroxy-4-(piperidin-1-yl)phenyl]-propionaldehyd-diethylacetal als Oel. IR ($CCl_4$): 3600 $cm^{-1}$ (OH).

Beispiel 9:

Eine Lösung von 3,23 g (10,0 mMol) 2-[2-Acetoxy-5-chlor-4-(piperidin--1-yl)phenyl]-1-methoxy-propen in 40 ml Tetrahydrofuran wird mit 0,5 g 10 proz. Palladium-Kohle versetzt und während 20 Stunden bei Raumtemperatur hydriert. Anschliessend wird vom Katalysator abfiltriert und im Vakuum eingedampft. Destillation (120-130°/0,01 Torr) ergibt 2-[2-Acetoxy-5-chlor-4-(piperidin-1-yl)phenyl]-1-methoxy-propan.

Das Ausgangsmaterial kann beispielsweise wie folgt hergestellt werden:

Ein Gemisch aus 18,9 g (55 mMol) Methoxymethyl-triphenyl-phosphoniumchlorid, 9,76 g (33 mMol) 2-Acetoxy-5-chlor-4-(piperidin-1-yl)-acetophenon und 2,70 g (50 mMol) Natriummethylat in 60 ml abs. Methanol wird während 50 Stunden bei 50° gehalten. Es wird genutscht und das

Methanol am Vakuumrotationsverdampfer entfernt. Der Rückstand wird in Ether/Petrolether aufgenommen. Das ausgefallene Triphenylphosphinoxid wird abgenutscht. Das Filtrat wird am Vakuumrotationsverdampfer eingeengt und anschliessend destilliert. (Sdp. 110-120°/0,01 Torr). So wird 2-[2-Acetoxy-5-chlor-4-(piperidin-1-yl)phenyl]-1-methoxy-propen erhalten.

Beispiel 10:

Eine Lösung von 2,82 g (10 mMol) 2-[5-Chlor-2-hydroxy-4-(piperidin-1-yl)phenyl]-1-methoxy-propen in 50 ml Tetrahydrofuran wird mit 0,6 g 10 proz. Palladium-Kohle versetzt und während 15 Stunden bei Raumtemperatur hydriert. Anschliessend wird vom Katalysator abfiltriert und im Vakuum eingedampft. Destillation (Sdp. 120-130°/0,01 Torr) ergibt 2-[5-Chlor-2-hydroxy-4-(piperidin-1-yl)phenyl]-1-methoxy-propan.

Das Ausgangsmaterial kann beispielsweise wie folgt hergestellt werden:

Eine Lösung von 3,23 g (10,0 mMol) 2-[2-Acetoxy-5-chlor-4-(piperidin-1-yl)phenyl]-1-methoxy-propen in 30 ml Ethanol wird mit 10 ml einer 2N Natronlauge-Lösung versetzt und während 14 Stunden bei Raumtemperatur gerührt. Das Ethanol wird am Vakuumrotationsverdampfer entfernt, der Rückstand mit Wasser verdünnt, mit Salzsäure neutralisiert und mit Methylenchlorid dreimal extrahiert. Das nach dem Trocknen und Eindampfen der oganischen Phasen erhaltene Rohprodukt wird destilliert (Sdp. 120-130°/0,01 Torr). Es resultiert 2-[5-Chlor-2-hydroxy-4-(piperidin-1-yl)phenyl]-1-methoxy-propen.

Beispiel 11:

Eine Lösung von 3,25 g (10,0 mMol) 2-[2-Acetoxy-5-chlor-4-(piperidin-1-yl)phenyl]-1-methoxy-propan in 30 ml Ethanol wird mit 10 ml einer 2N Natronlauge-Lösung versetzt und während 14 Stunden bei Raumtemperatur gerührt. Das Ethanol wird am Vakuumrotationsverdampfer entfernt, der Rückstand mit Wasser verdünnt, mit Salzsäure neutralisiert und mit Methylenchlorid dreimal extrahiert. Das nach dem

Trocknen und Eindampfen der organischen Phasen erhaltene Rohprodukt wird destilliert (Sdp. 120-130°/0,01 Torr). Es resultiert 2-[5-Chlor-2-hydroxy-4-(piperidin-1-yl)phenyl]-1-methoxy-propan.

Beispiel 12:

Ein Gemisch von 3,25 g (10,0 mMol) 2-[2-Acetoxy-5-chlor-4-(piperidin--1-yl)phenyl]-1-methoxy-propan in 30 ml Iodwasserstoffsäure wird während 4 Stunden unter Rückfluss gekocht. Das Reaktionsgemisch wird abgekühlt, mit Wasser verdünnt und dreimal mit Methylenchlorid extrahiert. Die organischen Phasen werden mit Wasser gewaschen, vereinigt, über Natriumsulfat getrocknet und am Vakuumrotationsverdampfer eingeengt. Destillation des Rückstandes (Sdp. 120-130°/0,02 Torr) liefert 2-[5-Chlor-2-hydroxy-4-(piperidin-1-yl)phenyl]-propanol.

Beispiel 13:

Zu einer Lösung von 1,89 g (50 mMol) Natriumborhydrid und 11,75 g (40 mMol) 2-[2-Acetoxy-5-chlor-4-(piperidin-1-yl)phenyl]-propen in 50 ml Diglyme werden unter Kühlung auf 0°C und unter Stickstoffatmosphäre 10,6 g (75 mMol) Bortrifluoridetherat in 10 ml Diglyme getropft. Anschliessend wird während 12 Stunden bei Raumtemperatur gerührt. Die Lösung wird auf 0° gekühlt und vorsichtig mit 15 ml Wasser versetzt. Dann werden nacheinander 40 ml 10 proz. Natronlauge-Lösung und 30 ml 30%ige Wasserstoffperoxid-Lösung zugetropft. Das Reaktionsgemisch wird anschliessend während 6 Stunden bei Raumtemperatur gerührt, mit viel Wasser verdünnt und mit Ether mehrmals extrahiert. Die Etherphasen werden mit Wasser gewaschen, vereinigt, über Natriumsulfat getrocknet und am Vakuumrotationsverdampfer eingeengt. Der Rückstand wird destilliert (Sdp. 120-130°/0,02 Torr). Man erhält so das 2-[5-Chlor-2-hydroxy-4-(piperidin-1-yl)phenyl]-propanol.

Das Ausgangsmaterial kann beispielsweise wie folgt hergestellt werden:

Zu einer frisch hergestellten Grignard-Lösung aus 4,86 g (0,20 Mol) Magnesium-Spänen und 28,4 g (0,20 Mol) Methyliodid in 300 ml abs. Ether wird bei Raumtemperatur eine Lösung von 59,2 g (0,20 Mol) 2-Acetoxy-5-chlor-4-(piperidin-1-yl)-acetophenon in 500 ml abs. Ether getropft. Anschliessend wird während 45 Minuten unter Rückfluss gekocht und auf Eis/Ammoniumchlorid gegossen. Die Etherphase wird abgetrennt und die wässrige Phase noch zweimal mit Ether extrahiert. Die organischen Phasen werden mit Wasser gewaschen, vereinigt, über Natriumsulfat getrocknet und am Vakuumrotationsverdampfer eingeengt. Der Rückstand wird mit 3,0 g Kaliumhydrogensulfit versetzt und destilliert. (Sdp. 110-120°/0,02 Torr). Man erhält so das 2-[2-Acetoxy-5-chlor-4-(piperidin-1-yl)phenyl]-propen.

Beispiel 14:

Ein Gemisch von 2,70 g (10,0 mMol) 2-[5-Chlor-2-hydroxy-4-(piperidin-1-yl)phenyl]-propanol und 40 ml Essigsäureanhydrid wird während 3 Stunden unter Rückfluss gekocht. Das Reaktionsgemisch wird am Vakuumrotationsverdampfer eingeengt und der Rückstand zwischen Methylenchlorid und verdünnter Bicarbonat-Lösung verteilt. Das nach dem Trocknen und Einengen der organischen Phasen erhaltene Rohprodukt wird mit Petrolether/Methylenchlorid am Kieselgel chromatographiert. Anschliessende Destillation (Sdp. 130-140°/0,03 Torr) ergibt 1-Acetoxy-2-[2-acetoxy-5-chlor-4-(piperidin-1-yl)phenyl]-propan.

Beispiel 15:

Zu einer Suspension von 1,6 g (42 mMol) Lithiumaluminiumhydrid in 90 ml abs. Tetrahydrofuran wird während ca. 15 Minuten eine Lösung von 14,2 g (50 mMol) 5-Chlor-2-methoxy-4-(piperidin-1-yl)-phenylessigsäure in 50 ml abs. Tetrahydrofuran getropft. Anschliessend wird noch 2 Stunden bei 50° nachgerührt. Nach Zugabe von 50 ml Wasser wird über Hyflo filtriert und mit THF nachgewaschen. Das Lösungsmittel wird am Vakuumrotationsverdampfer eingeengt und der Rückstand zwischen Ether und

Wasser verteilt. Die Etherphasen werden vereinigt, über Natriumsulfat getrocknet und am Vakuumrotationsverdampfer eingeengt. Der Rückstand wird an Kieselgel mit dem Laufmittel Chloroform/Essigester (1:1) chromatographiert. Umkristallisation aus Ether/Petrolether ergibt 2-[5-Chlor-2-methoxy-4-(piperidin-1-yl)phenyl]-ethanol vom Smp. 56-57°.

Das Ausgangsmaterial kann wie folgt hergestellt werden:

Eine Lösung von 96 g (0,72 Mol) Aluminiumtrichlorid in 180 ml abs. Nitromethan wird unter Stickstoff-Atmosphäre und Eis/Methanol-Kühlung zu einem Gemisch von 106,2 g (0,60 Mol) 3,4-Dichloranisol [H. Jamarlik et al, Comptes Rendus Acad. Sci. Ser. C 273 (25), 1756 (1971)] und 51,1 ml (0,72 Mol) Acetylchlorid während ca. 30 Minuten so zugetropft, dass der Innentemperaturbereich zwischen 0 und 5°C liegt. Anschliessend wird noch 1 Stunde bei etwa 4 bis 6°C weitergerührt, dann auf Eis gegossen und mit Methylenchlorid extrahiert. Die organischen Extrakte werden mit Wasser gewaschen, vereinigt, über Natriumsulfat getrocknet und am Vakuumrotationsverdampfer eingedampft. Nach Umkristallisation aus Methanol/Wasser erhält man das 4,5-Dichlor-2-methoxy-acetophenon vom Schmelzpunkt 93 bis 95°C.

Eine Lösung von 76,7 g (0,35 Mol) 4,5-Dichlor-2-methoxy-acetophenon in 750 ml Piperidin wird während 7 Stunden bei 170° C im Autoklaven gehalten. Das Reaktionsgemisch wird eingedampft, in Essigester aufgenommen und mit Wasser gewaschen. Die Essigester-Extrakte werden vereinigt, über Natriumsulfat getrocknet und am Vakuumrotationsverdampfer eingedampft. Der Rückstand wird an Kieselgel mit Methylenchlorid chromatographiert. Man erhält so das 5-Chlor-2-hydroxy-4-(N-piperidino)-acetophenon vom Schmelzpunkt 68 bis 70° C.

Eine Lösung von 32,5 g (128 mMol) 5-Chlor-2-hydroxy-4-(N-piperidino)-acetophenon und 75 ml (166 mMol) einer etwa 40 %igen methanolischen Lösung von Benzyltriethylammoniumhydroxid (Triton B) in 65 ml Tetrahydrofuran wird auf 0° C abgekühlt. Währen d ca. 6 Minuten werden 14,6 ml (154 mMol) Dimethylsulfat so zugetropft, dass die Innentemperatur nicht über 5° C steigt. Das Reaktionsgemisch wird noch 1 Stunde bei 0° gerührt, dann während etwa 30 Minuten unter Rückfluss gekocht. Das Reaktionsgemisch wird nun auf 400 ml Wasser gegossen und mit Essigester extrahiert. Die vereinigten Essigesterphasen werden mit Wasser gewaschen, über Natriumsulfat getrocknet und am Vakuumrotationsverdampfer eingedampft. Der Rückstand wird aus Methylenchlorid/Hexan umkristallisiert, und man erhält das 5-Chlor-2-methoxy-4-(N-piperidino)-acetophenon vom Schmelzpunkt 119 bis 120°C.

Eine Lösung von 18,2 g (68 mMol) 5-Chlor-2-methoxy-4-(piperidin-1-yl)-acetophenon und 4,36 g (136 mMol) Schwefel in 68 ml Morpholin wird während 5 Stunden bei 90° C gehalten. Das Reaktionsgemisch wird abgekühlt, mit Essigester verdünnt und mit Wasser gewaschen. Die vereinigten Essigester-Extrakte werden über Natriumsulfat getrocknet und am Vakuumrotationsverdampfer eingedampft. Nach Umkristallisation aus Methylenchlorid/Methanol erhält man das 5-Chlor-2-methoxy-4-(piperidin-1-yl)-phenylthioessigsäuremorpholinamid vom Schmelzpunkt 137 bis 139°C.

Eine Lösung von 11,07 g (30 mMol) 5-Chlor-2-methoxy-4-(piperidin-1-yl)-phenylthioessigsäuremorpholinamid in 120 ml Eisessig und 30 ml konz. Salzsäure wird während 22 Stunden unter Rückfluss gekocht. Das Reaktionsgemisch wird abgekühlt, mit Wasser verdünnt und mit Methylenchlorid extrahiert. Die vereinigten Methylenchloridphasen werden mit Wasser gewaschen, über Natriumsulfat getrocknet und am Hochvakuumrotationsverdampfer eingedampft. Nach Chromatographieren an Kieselgel mit Chloroform/Methanol (19:1) erhält man die 5-Chlor-2-methoxy-4-(piperidin-1-yl)-phenylessigsäure, die nach Umkristallisation mit Methylenchlorid/Hexan bei 120 bis 122° C schmilzt.

Beispiel 16:

Eine Lösung von 13,5 g (50 mMol) 2-[5-Chlor-2-methoxy-4-(pipiridin-1-yl)-phenyl]-ethanol in 150 ml abs. Pyridin wird mit 6,12 g (60 mMol) Essigsäureanhydrid versetzt und über Nacht gerührt. Anschliessend wird am Vakuumrotationsverdampfer eingeengt und der Rückstand in Methylenchlorid aufgenommen, mit verdünnter Salzsäure und Wasser gewaschen. Die organischen Phasen werden vereinigt, über Natriumsulfat getrocknet und am Vakuumrotationsverdampfer eingeengt. Destillation (120-130°/0,01 Torr) ergibt 1-Acetoxy-2-[5-chlor-2-methoxy-4-(piperidin-1-yl)phenyl]-ethan.

Beispiel 17:

Zu einer auf -70° gekühlte Lösung von 3,1 g (10 mMol) 1-Acetoxy-2-[5-chlor-2-methoxy-4-(piperidin-1-yl)phenyl]-ethan in 70 ml abs. Methylenchlorid werden während ca. einer Minute 1,9 ml (20 mMol) Bortribromid getropft. Nach kurzem Erwärmen auf Raumtemperatur wird wieder auf -70° gekühlt und mit Wasser hydrolisiert. Das Kühlbad wird entfernt und die organische Phase abgetrennt. Die Wasserphase wird noch zweimal mit Methylenchlorid nachextrahiert. Die organischen Auszüge werden mit Wasser gewaschen, vereinigt, über Natriumsulfat getrocknet und am Vakuumrotationsverdampfer eingeengt. Destillation (120-130°/0,015 Torr) liefert 1-Acetoxy-2-[5-chlor-2-hydroxy-4-(piperidin-1-yl)phenyl]-ethan.

Beispiel 18:

Eine Lösung von 2,97 g (10,0 mMol) 1-Acetoxy-2-[5-chlor-2-hydroxy-4-(piperidin-1-yl)phenyl]-ethan in 30 ml Ethanol wird mit 10 ml einer 2N Natronlauge-Lösung versetzt und während 14 Stunden bei Raumtemperatur gerührt. Das Ethanol wird am Vakuumrotationsverdampfer entfernt, der Rückstand mit Wasser verdünnt, mit Salzsäure neutralisiert und mit Methylenchlorid dreimal extrahiert. Das nach dem Trocknen und Eindampfen der organischen Phasen erhaltene Rohprodukt wird destilliert

(Sdp. 120-130°/0,02 Torr). Es resultiert 2-[5-Chlor-2-hydroxy-4-(piperidin-1-yl)phenyl]-ethanol.

Beispiel 19:

Eine Lösung von 8,8 g (0,20 Mol) Ethylenoxid in 30 ml abs. Tetrahydrofuran wird langsam unter Eis/Wasser-Kühlung zu einer Lösung von 2-Acetoxy-5--methyl-4-(pyrrol-1-yl)phenylmagnesiumbromid [hergestellt aus 58,8 g (0,20 Mol) 2-Acetoxy-5-methyl-4-(pyrrol-1-yl)-brombenzol und 4,86 g (0,20 Mol) Magnesium Spänen in 45 ml abs. Tetrahydrofuran] getropft. Anschliessend wird noch eine Stunde unter Rückfluss gekocht. Das Reaktionsgemisch wird abgekühlt und mit verdünnter Salzsäure vorsichtig hydrolisiert. Das Tetrahydrofuran wird am Vakuumrotationsverdampfer entfernt und die wässrige Phase dreimal mit Methylenchlorid extrahiert. Die organischen Phasen werden neutral gewaschen, vereinigt, über Natriumsulfat getrocknet und am Vakuumrotationsverdampfer eingeengt. Destillation des Rückstands im Kugelrohr (120-130°/0,03 Torr) ergibt 2-[2-Acetoxy-5-methyl-4-(pyrrol-1-yl)-phenyl]-ethanol.

Das Ausgangsmaterial kann beispielsweise wie folgt hergestellt werden:

109,6 g (0,40 Mol) 2-Acetoxy-5-methyl-4-nitro-brombenzol (W.O. Kermack et al, J.C.S. 1932, 2946) werden in 1,3 l Tetrahydrofuran aufgenommen, mit 40 g Raney-Nickel versetzt und während 8 Stunden bei Raumtemperatur hydriert. Anschliessend wird vom Katalysator abfiltriert und eingedampft. Kurzwegdestillation bei 150-170°/0,02 Torr ergibt 2-Acetoxy-4-amino-5-methyl-brombenzol.

78,1 g (0,32 Mol) 2-Acetoxy-4-amino-5-methyl-brombenzol in 700 ml Eisessig werden unter Rühren bei 104-106° innert 15 Minuten mit 58 g (0,44 Mol) 2,5-Dimethoxytetrahydrofuran versetzt. Nach 20 Minuten wird rasch abgekühlt und der Eisessig am Vakuumrotationsverdampfer eingeengt. Der Rückstand wird im Methylenchlorid aufgenommen und dreimal mit Wasser gewaschen. Das nach dem Trocknen und Eindampfen der

organischen Phasen erhaltene Rohprodukt wird mit Petrolether/Ether an Kieselgel chromatographiert. Anschliessende Destillation der reinen Fraktionen im Kugelrohr (110-120°/0,04 Torr) ergibt 2-Acetoxy-5-methyl-4-(pyrrol-1-yl)-brombenzol.

Beispiel 20:

Zu einer Suspension von 0,8 g (20 mMol) Lithiumaluminiumhydrid in 50 ml abs. Ether werden unter Stickstoffatmosphäre, Rühren und Eiskühlung bei 0-5° innert 30 Minuten 3,57 g (10,0 mMol) 3,5-Dimethyl-6-dibenzylamino-benzofuran-2(3H)-on gelöst in 100 ml abs. Ether getropft. Anschliessend wird 3 Stunden bei Raumtemperatur gerührt. Durch vorsichtiges Zutropfen von ca. 10 ml Wasser unter Eiskühlung wird der Lithiumaluminiumkomplex zerlegt. Mittels 1N Salzsäure-Lösung wird schwach angesäuert und 5 mal mit Methylenchlorid extrahiert. Die organischen Phasen werden vereinigt, über Natriumsulfat getrocknet und am Vakuumrotationsverdampfer eingeengt. Man erhält so 2-[4-Dibenzylamino-2-hydroxy-5-methyl-phenyl]-propanol.

Beispiel 21:

Ein Gemisch von 3,61 g (10,0 mMol) rohem 2-[4-Dibenzylamino-2-hydroxy-5-methyl-phenyl]-propanol und 40 ml Pivalinsäureanhydrid wird während 3 Stunden bei 120° gehalten. Das Reaktionsgemisch wird abgekühlt, mit Wasser vorsichtig hydrolisiert und dann dreimal mit Methylenchlorid extrahiert. Die organischen Phasen werden mit verdünnter Natriumbicarbonat-Lösung gewaschen, vereinigt, über Natriumsulfat getrocknet und am Vakuumrotationsverdampfer eingeengt. Der Rückstand wird mit Petrolether/Methylenchlorid am Kieselgel chromatographiert. Man erhält so 2-[4-Dibenzylamino-5-methyl-2-pivaloyloxy-phenyl]-1-pivaloyloxy-propan als Oel.

Beispiel 22:

Ein Gemisch von 3,50 g (10,0 mMol) rohem 2-[4-Amino-5-methyl-2-pivaloyloxy-phenyl]-1-pivaloyloxy-propan in 50 ml Eisessig wird unter Rühren bei 104-106° innert 5 Minuten mit 1,95 g (15,0 mMol) 2,5-Dimethoxytetrahydrofuran versetzt. Nach weiteren 5 Minuten wird rasch abgekühlt und der Eisessig am Vakuumrotationsverdampfer eingeengt. Der Rückstand wird in Methylenchlorid aufgenommen und dreimal mit Wasser gewaschen. Das nach dem Trocknen und Eindampfen der organischen Phasen erhaltene Rohprodukt wird mit Petrolether/Ether an Kieselgel chromatographiert. Anschliessende Destillation der reinen Fraktionen im Kugelrohr (180-190°/0,05 Torr) ergibt 2-[5-Methyl-2-pivaloyloxy-4-(pyrrol-1-yl)phenyl]-1-pivaloyloxy-propan.

Das Ausgangsmaterial kann beispielsweise wie folgt hergestellt werden:

Eine Lösung von 5,30 g (10,0 mMol) 2-[4-Dibenzylamino-5-methyl-2-pivaloyloxy-phenyl]-1-pivaloyloxy-propan in 30 ml Dioxan wird mit 0,6 g 10 proz. Palladium-Kohle versetzt und während 20 Stunden bei Raumtemperatur hydriert. Anschliessend wird vom Katalysator abfiltriert und am Vakuumrotationsverdampfer eingeengt. Man erhält so 2-[4-Amino-5-methyl-2-pivaloyloxy-phenyl]-1-pivaloyloxy-propan, das ohne weitere Reinigung in die nächste Stufe eingesetzt wird.

Beispiel 23:

Ein Lösung von 4,00 g (10,0 mMol) 2-[5-Methyl-2-pivaloyloxy-4-(pyrrol-1-yl)phenyl]-1-pivaloyloxy-propan in 30 ml Ethanol wird mit 10 ml einer 3N Natronlauge-Lösung versetzt und während 15 Stunden bei Raumtemperatur gerührt. Das Ethanol wird am Vakuumrotationsverdampfer entfernt, der Rückstand mit Wasser verdünnt, mit verdünnter Salzsäure neutralisiert und mit Methylenchlorid dreimal extrahiert. Die organischen Phasen werden vereinigt, über Natriumsulfat getrocknet und am Vakuumrotationsverdampfer eingeengt. Umkristallisation aus Diisopropylether liefert 2-[2-Hydroxy-5-methyl-4-(pyrrol-1-yl)phenyl]-propanol vom Smp. 117-118°.

Beispiel 24:

Tabletten, enthaltend 25 mg Wirkstoff, z.B. 2-[5-Chlor-2-hydroxy-4-(piperidin-1-yl)phenyl]-propan-1-ol, können folgendermassen hergestellt werden:

Bestandteile (für 1000 Tabletten):

| | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke wird in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35°C getrocknet, durch ein Sieb mit 1,2 mm Maschenweite getrieben und zu beidseitig konkaven Tabletten von etwa 6 mm Durchmesser verpresst.

Beispiel 25:

Kautabletten enthaltend 30 mg Wirkstoff, z.B. 2-[5-Chlor-2-hydroxy-4-(piperidin-1-yl)phenyl]-propan-1-ol, können z.B. folgendermassen hergestellt werden:

Zusammensetzung (für 1000 Tabletten):

| | |
|---|---|
| Wirkstoff | 30,0 g |
| Mannit | 267,0 g |
| Lactose | 179,5 g |
| Talkum | 20,0 g |
| Glycin | 12,5 g |
| Stearinsäure | 10,0 g |
| Saccharin | 1,0 g |
| 5%ige Gelatinelösung | q.s. |

Herstellung: Alle festen Ingredienzien werden zunächst durch ein Sieb mit 0,25 mm Maschenweite getrieben. Der Mannit und die Lactose werden gemischt, unter Hinzufügen von Gelatinelösung granuliert, durch ein Sieb mit 2 mm Maschenweite getrieben, bei 50°C getrocknet und nochmals durch ein Sieb mit 1,7 mm Maschenweite getrieben. Der Wirkstoff, das Glycin und das Saccharin werden sorgfältig vermischt, der Mannit, das Lactosegranulat, die Stearinsäure und das Talkum hinzugegeben, das Ganze gründlich vermischt und zu beidseitig konkaven Tabletten von etwa 100 mm Durchmesser mit Bruchrille auf der Oberseite verpresst.

Beispiel 26:

Tabletten enthaltend 100 mg Wirkstoff, z.B. 2-[5-Methyl-2-hydroxy-(4-pyrrol-1-yl)-phenyl]-propan-1-ol, können folgendermassen hergestellt werden:

Zusammensetzung (für 1000 Tabletten):

| | |
|---|---|
| Wirkstoff | 100,0 g |
| Lactose | 248,5 g |
| Maisstärke | 17,5 g |
| Polyethylenglykol 6000 | 5,0 g |
| Talkum | 15,0 g |
| Magnesiumstearat | 4,0 g |
| entmineralisiertes Wasser | q.s. |

Herstellung: Die festen Ingredienzien werden zunächst durch ein Sieb mit 0,6 mm Maschenweite getrieben. Dann werden Wirkstoff, Lactose, Talkum, Magnesiumstearat und die Hälfte der Stärke innig vermischt. Die andere Hälfte der Stärke wird in 65 ml Wasser suspendiert und diese Suspension zu einer siedenden Lösung des Polyethylenglykols in 260 ml Wasser hinzugegeben. Der erhaltene Kleister wird zu den pulverförmigen Substanzen hinzugefügt, das Ganze vermischt und granuliert, erforderlichenfalls unter Zugabe von Wasser. Das Granulat wird über Nacht bei 35°C getrocknet, durch ein Sieb mit 1,2 mm Maschenweite getrieben und zu beidseitig konkaven Tabletten von etwa 10 mm Durchmesser mit Bruchkerbe auf der Oberseite verpresst.

In analoger Weise, wie in der Beispielen 22 bis 24 beschrieben, können sämtliche erfindungsgemässen Verbindungen formuliert werden.

BAD ORIGINAL

Patentansprüche für die Vertragsstaaten: GB, BE, DE, FR, IT, LU, NL, SE, CH

1. Phenol-Derivate der allgemeinen Formel

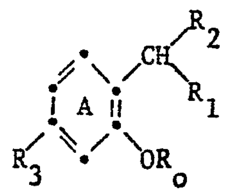

(I),

worin $R_o$ Wasserstoff oder einen Acylrest darstellt, $R_1$ Hydroxymethyl, verestertes oder verethertes Hydroxymethyl, Formyl oder acetalisiertes Formyl bedeutet, $R_2$ für Wasserstoff oder einen aliphatischen Rest steht, $R_3$ eine durch zwei einwertige Kohlenwasserstoffreste oder durch einen zweiwertigen, gegebenenfalls durch mindestens ein Heteroatom unterbrochenen Kohlenwasserstoffrest disubstituierte Aminogruppe darstellt und der aromatische Ring A zusätzlich substituiert sein kann, und ihre Salze und Isomeren.

2. Verbindungen gemäss Anspruch 1 der Formel I, worin $R_o$ Wasserstoff, einen Niederalkanoyl- oder einen Arylniederalkanoylrest bedeutet, $R_1$ Hydroxymethyl, mit einer Carbonsäure verestertes oder mit einem Alkohol verethertes Hydroxymethyl, Formyl oder mit einem Alkohol acetalisiertes Formyl, darstellt, $R_2$ einen gesättigten und unsubstituierten aliphatischen Rest bedeutet, $R_3$ eine durch zwei einwertige aliphatische Reste disubstituierte Aminogruppe oder eine durch einen zweiwertigen, gegebenenfalls durch Aza, N-Niederalkylaza, Oxa oder Thia unterbrochenen aliphatischen Rest disubstituierte Aminogruppe darstellt und der aromatische Ring A zusätzlich durch einen aliphatischen Rest, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_3$, unsubstituiert sein kann, und ihre Salze und Isomeren.

3. Verbindungen gemäss Anspruch 1 der Formel I, worin $R_o$ Wasserstoff, Niederalkanoyl oder Phenylniederalkanoyl bedeutet, wobei der Phenylrest ein- oder mehrfach durch Niederalkyl, Hydroxyniederalkyl, Halogenniederalkyl, Niederalkenyl, 3- oder 4-gliedriges Niederalkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, $R_1$ Hydroxymethyl, mit einer gegebenenfalls substituierten Niederalkancarbonsäure verestertes oder mit einem Niederalkanol verethertes Hydroxymethyl, Formyl oder mit einem Niederalkanol acetalisiertes Formyl darstellt, $R_2$ Wasserstoff oder Niederalkyl bedeutet und $R_3$ einerseits N,N-Di-niederalkyl-amino, N-Cycloniederalkyl-N-niederalkyl-amino, N-Niederalkyl-N-phenylniederalkyl-amino, N,N-Di-cycloniederalkylniederalkyl-amino, N-Cycloniederalkyl-N-phenylniederalkyl-amino oder N,N-Di-phenylniederalkyl-amino bedeutet oder $R_3$ andererseits jeweils 5- bis 8-gliedriges Niederalkylenamino, Niederalkenylenamino, durch Monoaza, N'-Niederalkylmonoaza, Monooxa oder Monothia unterbrochenes Niederalkylenamino, durch Monoaza, N'-Niederalkylmonoaza, Monooxa oder Monothia unterbrochenes Niederalkenylenamino oder ein oder zwei ortho-anellierte Benzosysteme aufweisendes Niederalkylen- bzw. Niederalkenylen-amino darstellt, wobei Niederalkylen bzw. Niederalkenylen unverzweigt oder verzweigt sein und 4 bis 14, insbesondere 4 bis 7, C-Atome aufweisen kann und/oder ein oder zwei ortho-anellierte Benzosysteme aufweisen kann und Phenyl oder Benzo jeweils ein- oder mehrfach durch Niederalkyl, Hydroxyniederalkyl, Halogenniederalkyl, Niederalkenyl, 3- oder 4-gliedriges Niederalkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, und der aromatische Ring A ein- oder mehrfach durch Niederalkyl, Hydroxyniederalkyl, Halogenniederalkyl, Niederalkenyl, 3- oder 4-gliedriges Niederalkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder, bis auf $R_3$, unsubstituiert sein kann, und ihre Salze und Isomeren.

4. Verbindungen gemäss Anspruch 1 der Formel

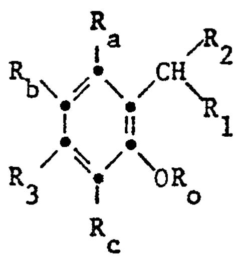

(Ia),

worin $R_o$ Wasserstoff oder Niederalkanoyl bedeutet,

$R_1$ Hydroxymethyl, Niederalkanoyloxymethyl, Niederalkoxymethyl, Formyl oder Diniederalkoxymethyl darstellt,

$R_2$ Wasserstoff oder Niederalkyl bedeutet,

$R_3$ einerseits N,N-Di-phenylniederalkyl-amino darstellt oder $R_3$ andererseits jeweils 5- bis 8-gliedriges Niederalkylenamino, durch Monooxa unterbrochenes Niederalkylenamino, oder ein ortho-anelliertes Benzosystem aufweisendes Niederalkylenamino bzw. Niederalkenylenamino bedeutet, und/oder $R_a$, $R_b$ und $R_c$ unabhängig voneinander Wasserstoff, Niederalkyl oder Halogen darstellen, und ihre Salze und Isomeren.

5. Verbindungen gemäss Anspruch 1 der Formel Ia, worin $R_o$ Wasserstoff oder Niederalkanoyl bedeutet, $R_1$ Hydroxymethyl, Niederalkanoyloxymethyl, Niederalkoxymethyl, Formyl oder Diniederalkoxymethyl darstellt, $R_2$ Wasserstoff oder Niederalkyl darstellt, $R_3$ Di-niederalkyl-amino, 5- bis 8-gliedriges Niederalkylenamino, 5- bis 8-gliedriges Niederalkenylenamino oder 5- bis 8-gliedriges Monooxa-niederalkylenamino bedeutet und $R_a$ sowie $R_c$ Wasserstoff bedeuten und $R_b$ Halogen bedeutet, und ihre Salze und Isomeren.

6. Verbindungen gemäss Anspruch 1 der Formel Ia, worin $R_o$ Wasserstoff oder Niederalkanoyl mit bis und mit 5 C-Atomen, bedeutet, $R_1$ Hydroxymethyl, Niederalkanoyloxymethyl, Niederalkoxymethyl, Formyl oder Diniederalkoxymethyl darstellt, $R_2$ Niederalkyl mit bis und mit 4 C-Atomen bedeutet, $R_3$ 5- bis 7-gliedriges Niederalkylenamino., Morpholin-4-yl oder Pyrrol-1-yl darstellt, $R_a$ und $R_c$ jeweils Wasserstoff bedeuten und $R_b$ Niederalkyl mit bis und mit 4 C-Atomen oder Halogen bis und mit Atomnummer 35 darstellt, und ihre Salze und Isomeren.

7. Verbindungen gemäss Anspruch 1 der Formel Ia, worin $R_o$ Niederalkanoyl mit bis und mit 5 C-Atomen darstellt, $R_1$ Hydroxymethyl, Niederalkoxymethyl mit bis und mit 5 C-Atomen oder Formyl bedeutet, $R_2$ Niederalkyl mit bis und mit 4 C-Atomen darstellt, $R_3$ Morpholin-4-yl oder Pyrrol-1-yl ist, $R_a$ und $R_c$ Wasserstoff bedeuten und $R_b$ Halogen bis und mit Atomnummer 35 oder Niederalkyl mit bis und mit 4 C-Atomen darstellt,und ihre Salze und Isomeren.

8. Verbindungen gemäss Anspruch der Formel Ia, worin $R_o$ Wasserstoff oder Niederalkanoyl mit bis und mit 5 C-Atomen darstellt, $R_1$ Hydroxymethyl bedeutet, $R_2$ Niederalkyl mit bis und mit 4 C-Atomen darstellt, $R_3$ 5- bis 7-gliedriges Niederalkylenamino, Morpholin-4-yl oder Pyrrol-1-yl ist, $R_a$ und $R_c$ Wasserstoff bedeuten und $R_b$ Halogen bis und mit Atomnummer 35 oder Niederalkyl mit bis und mit 4 C-Atomen darstellt, und ihre Salze und Isomeren.

9. Verbindungen gemäss Anspruch 1, der Formel Ia, worin $R_o$ Wasserstoff oder Niederalkanoyl mit bis und mit 5 C-Atomen darstellt, $R_1$ Formyl bedeutet, $R_2$ Niederalkyl mit bis und mit 4 C-Atomen darstellt, $R_3$ 5- bis 7-gliedriges Niederalkylenamino, Morpholin-4-yl oder Pyrrol-1-yl ist, $R_a$ und $R_c$ Wasserstoff bedeuten und $R_b$ Halogen bis und mit Atomnummer 35 oder Niederalkyl mit bis und mit 4 C-Atomen darstellt, und ihre Salze und Isomeren.

10. Verbindungen gemäss Anspruch 1, der Formel Ia, worin $R_o$ Wasserstoff oder Niederalkanoyl mit bis und mit 5 C-Atomen bedeutet, $R_1$ Hydroxymethyl, Niederalkanoyloxymethyl, Niederalkoxymethyl, Formyl oder Diniederalkoxymethyl darstellt, $R_2$ Niederalkyl mit bis und mit 4 C-Atomen bedeutet, $R_3$ Pyrrol-1-yl darstellt, $R_a$ und $R_c$ jeweils Wasserstoff bedeuten und $R_b$ Niederalkyl mit bis und mit 4 C-Atomen oder Halogen bis und mit Atomnummer 35 darstellt, und ihre Salze und Isomeren.

11. Verbindungen gemäss Anspruch 1, der Formel Ia, worin $R_o$, $R_a$ und $R_c$ jeweils Wasserstoff bedeuten, $R_b$ und $R_2$ jeweils Niederalkyl mit bis und mit 4 C-Atomen, wie Methyl, darstellen, $R_1$ Hydroxymethyl ist, und $R_3$ Pyrrol-1-yl bedeutet.

12. 2-(5-Chlor-2-hydroxy-4-morpholino-phenyl)-propan-1-ol.

13. 2-[2-Hydroxy-5-methyl-4-(pyrrol-1-yl)-phenyl]-propan-1-ol.

14. 2-[2-Hydroxy-5-methyl-4-(pyrrol-1-yl)-phenyl]-propanal oder ein Isomeres davon.

15. 2-[5-Chlor-2-hydroxy-4-(piperidin-1-yl)-phenyl]-propanol, 2-[2-Acetoxy-5-chlor-4-(piperidin-1-yl)-phenyl]-propionaldehyd, 2-[2-Acetoxy-5-chlor-4-(piperidin-1-yl)-phenyl]-propanol, 2-[2-Acetoxy-5-chlor-4-(piperidin-1-yl)-phenyl]-propionaldehyd-diethylacetal, 2-[5-Chlor-2-hydroxy-4-(piperidin-1-yl)-phenyl]-propionaldehyd-diethylacetal, 2-[2-Acetoxy-5-chlor-4-(piperidin-1-yl)-phenyl]-1-methoxy-propan, 2-[5-Chlor-2-hydroxy-4-(piperidin-1-yl)-phenyl]-1-methoxy-propan, 1-Acetoxy-2-[2-acetoxy-5-chlor-4-(piperidin-1-yl)-phenyl]-propan, 1-Acetoxy-2-[5-chlor-2-hydroxy-4-(piperidin-1-yl)-phenyl]-ethan, 2-[5-Chlor-2-hydroxy-4-(piperidin-1-yl)-phenyl]-ethanol, 2-[2-Acetoxy-5-methyl-4-(pyrrol-1-yl)-phenyl]-ethanol, 2-[4-Dibenzylamino-2-hydroxy-5-methyl-phenyl]-propanol, 2-[4-Dibenzylamino-5-methyl-2-pivaloyloxy-phenyl]-1-pivaloyloxypropan oder 2-[5-Methyl-2-pivaloyloxy-4-(pyrrol-1-yl)-phenyl]-1-pivaloyloxypropan.

16. Verbindung gemäss einem der Ansprüche 1-15 mit antiinflammatorischer und/oder analgetischer Wirkung.

17. Verbindung gemäss einem der Ansprüche 1-16 zur Verwendung in einem Verfahren zur therapeutischen Behandlung des menschlichen Körper.

18. Pharmazeutische Präparate, enthaltend mindestens eine Verbindung gemäss einem der Ansprüche 1-17.

19. Verwendung von Verbindungen gemäss einem der Ansprüche 1-17 zur Behandlung von entzündlicher Erkrankungen und/oder von Schmerzzuständen.

20. Verwendung von Verbindungen gemäss einem der Ansprüche 1-17 zur Herstellung von pharmazeutischen Präparaten.

21. Verfahren zur Herstellung pharmazeutischer Präparate, dadurch gekennzeichnet, dass man eine Verbindung gemäss einem der Ansprüche 1-17, gegebenenfalls unter Beimischung von üblichen Hilfs- und Trägerstoffen, zu pharmazeutischen Präparaten verarbeitet.

22. Verfahren zur Herstellung von Phenol-Derivaten der allgemeinen Formel 1

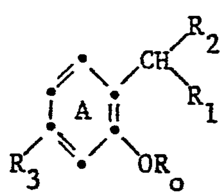

(I),

worin $R_o$ Wasserstoff oder einen Acylrest darstellt, $R_1$ Hydroxymethyl, verestertes oder verethertes Hydroxymethyl, Formyl oder acetalisiertes Formyl bedeutet, $R_2$ für Wasserstoff oder einen aliphatischen Rest steht, $R_3$ eine durch zwei einwertige Kohlenwasserstoffreste oder durch einen zweiwertigen, gegebenenfalls durch mindestens ein Heteroatom unterbrochenen Kohlenwasserstoffrest disubstituierte Aminogruppe darstellt und der aromatische Ring A zusätzlich substituiert sein kann, und ihre Salze und Isomeren, dadurch gekennzeichnet, dass man in einer Verbindung der Formel

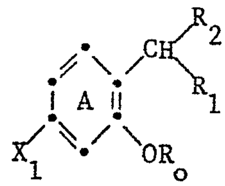

(II) ,

einem Salz oder Isomeren davon, worin $X_1$ für einen in $R_3$ überführbaren Rest steht, $X_1$ in $R_3$ überführt, oder in einer Verbindung der Formel

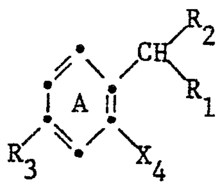

(III) ,

einem Salz oder Isomeren davon, worin $X_4$ für einen in die Gruppe $-OR_o$ überführbaren Rest steht, den Rest $X_4$ in die Gruppe $-OR_o$ überführt, oder in einer Verbindung der Formel

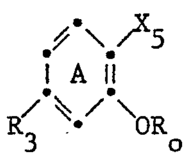

(IV)

oder einem Salz davon, worin $X_5$ für einen in die Gruppe der Formel $-CH(R_2)-R_1$ überführbaren Rest steht, $X_5$ in die Gruppe der Formel $-CH(R_2)-R_1$ überführt und gewünschtenfalls eine erhaltene erfindungsgemässe Verbindung in eine andere erfindungsgemässe Verbindung überführt und/oder gewünschtenfalls ein erhaltenes Salz in die freie erfindungsgemässe Verbindung oder in ein anderes erfindungsgemässes Salz überführt und/oder gewünschtenfalls eine erhaltene erfindungsgemässe Verbindung mit salzbildenden Eigenschaften in ein Salz überführt und/oder gewünschtenfalls ein erhaltenes Isomerengemisch in die einzelnen Komponenten auftrennt.

23. Verfahren gemäss Anspruch 22, dadurch gekennzeichnet, dass man in einer Verbindung der Formel

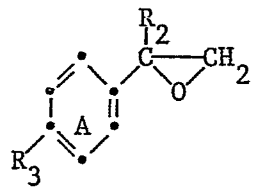

(IV")

den Oxiranring in Gegenwart einer Lewissäure zur Verbindung der Formel I öffnet, worin $R_1$ Formyl bedeutet und Formyl gewünschtenfalls in an sich bekannter Weise acetalisiert oder zu Hydroxymethyl $R_1$ und dieses wiederum verestert oder verethert.

24. Verfahren nach Anspruch 22, dadurch gekennzeichnet, dass man eine Verbindung der Formel

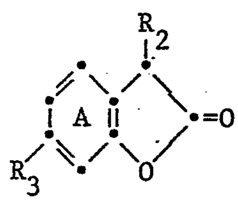

(IV')

mit Hilfe eines Reduktionsmittels zu einer Verbindung der Formel I reduziert, worin $R_1$ Hydroxymethyl ist.

25. Verfahren nach Anspruch 24, dadurch gekennzeichnet, dass man 5-Chlor-3-methyl-6-morpholino-benzofuran-2(3H)-on mit Lithiumaluminiumhydrid zu 2-(5-Chlor-2-hydroxy-4-morpholino-phenyl)-propan-1-ol reduziert.

26. Verfahren gemäss einem der Ansprüche 22-25, dadurch gekennzeichnet, dass man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

27. Die nach dem Verfahren gemäss einem der Ansprüche 22-25 erhältlichen Verbindungen.

Patentansprüche für den Vertragsstaat AT

1. Verfahren zur Herstellung von Phenol-Derivaten der allgemeinen Formel 1

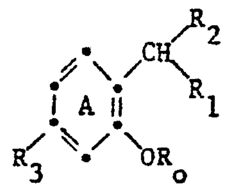

(I),

worin $R_o$ Wasserstoff oder einen Acylrest darstellt, $R_1$ Hydroxymethyl, verestertes oder verethertes Hydroxymethyl, Formyl oder acetalisiertes Formyl bedeutet, $R_2$ für Wasserstoff oder einen aliphatischen Rest steht, $R_3$ eine durch zwei einwertige Kohlenwasserstoffreste oder durch einen zweiwertigen, gegebenenfalls durch mindestens ein Heteroatom unterbrochenen Kohlenwasserstoffrest disubstituierte Aminogruppe darstellt und der aromatische Ring A zusätzlich substituiert sein kann, und ihre Salze und Isomeren, dadurch gekennzeichnet, dass man in einer Verbindung der Formel

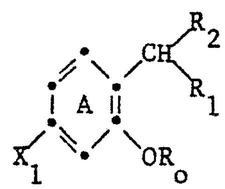

(II) ,

einem Salz oder Isomeren davon, worin $X_1$ für einen in $R_3$ überführbaren Rest steht, $X_1$ in $R_3$ überführt, oder in einer Verbindung der Formel

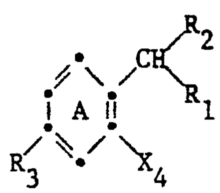

(III) ,

einem Salz oder Isomeren davon, worin $X_4$ für einen in die Gruppe $-OR_o$ überführbaren Rest steht, den Rest $X_4$ in die Gruppe $-OR_o$ überführt, oder in einer Verbindung der Formel

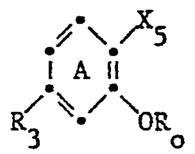

(IV)

oder einem Salz davon, worin $X_5$ für einen in die Gruppe der Formel $-CH(R_2)-R_1$ überführbaren Rest steht, $X_5$ in die Gruppe der Formel $-CH(R_2)-R_1$ überführt und gewünschtenfalls eine erhaltene erfindungsgemässe Verbindung in eine andere erfindungsgemässe Verbindung überführt und/oder gewünschtenfalls ein erhaltenes Salz in die freie erfindungsgemässe Verbindung oder in ein anderes erfindungsgemässes Salz überführt und/oder gewünschtenfalls eine erhaltene erfindungsgemässe Verbindung mit salzbildenden Eigenschaften in ein Salz überführt und/oder gewünschtenfalls ein erhaltenes Isomerengemisch in die einzelnen Komponenten auftrennt.

2. Verfahren gemäss Anspruch 1, dadurch gekennzeichnet, dass man in einer Verbindung der Formel

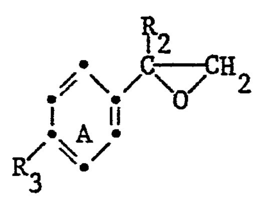

(IV")

den Oxiranring in Gegenwart einer Lewissäure zur Verbindung der Formel I öffnet, worin $R_1$ Formyl bedeutet und Formyl gewünschtenfalls in an sich bekannter Weise acetalisiert oder zu Hydroxymethyl $R_1$ und dieses wiederum verestert oder verethert.

3. Verfahren nach Anspruch 1, dadurch gekennzeichnet, dass man eine Verbindung der Formel

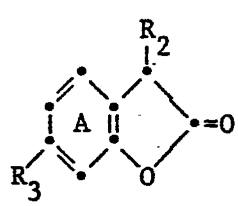

(IV')

mit Hilfe eines Reduktionsmittels zu einer Verbindung der Formel I reduziert, worin $R_1$ Hydroxymethyl ist.

4. Verfahren nach Anspruch 3, dadurch gekennzeichnet, dass man 5-Chlor-3-methyl-6-morpholino-benzofuran-2(3H)-on mit Lithiumaluminiumhydrid zu 2-(5-Chlor-2-hydroxy-4-morpholino-phenyl)-propan-1-ol reduziert.

5. Verfahren gemäss einem der Ansprüche 1-4, dadurch gekennzeichnet, dass man von einer auf irgendeiner Stufe des Verfahrens als Zwischenprodukt erhältlichen Verbindungen ausgeht und die fehlenden Schritte durchführt oder einen Ausgangsstoff in Form eines Salzes verwendet oder insbesondere unter den Reaktionsbedingungen bildet.

6. Verfahren nach einem der Ansprüche 1-5, dadurch gekennzeichnet, dass man eine Verbindung der Formel I, worin $R_o$ Wasserstoff, einen Niederalkanoyl- oder einen Arylniederalkanoylrest bedeutet, $R_1$ Hydroxymethyl, mit einer Carbonsäure verestertes oder mit einem Alkohol verethertes Hydroxymethyl, Formyl oder mit einem Alkohol acetalisiertes Formyl, darstellt, $R_2$ einen gesättigten und unsubstituierten aliphatischen Rest bedeutet, $R_3$ eine durch zwei einwertige aliphatische Reste disubstituierte Aminogruppe oder eine durch einen zweiwertigen, gegebenenfalls durch Aza, N-Niederalkylaza, Oxa oder Thia unterbrochenen aliphatischen Rest disubstituierte Aminogruppe darstellt und der aromatische Ring A zusätzlich durch einen aliphatischen Rest, Niederalkoxy, Niederalkylthio, Niederalkansulfinyl, Niederalkansulfonyl, Hydroxy, Halogen, Niederalkanoyloxy, Niederalkanoyl und/oder Nitro ein- oder mehrfach substituiert oder, bis auf $R_3$, unsubstituiert sein kann, oder ein Salz oder Isomeres davon herstellt.

7. Verfahren nach einem der Ansprüche 1-5, dadurch gekennzeichnet, dass man eine Verbindung der Formel I, worin $R_o$ Wasserstoff, Niederalkanoyl oder Phenylniederalkanoyl bedeutet, wobei der Phenylrest ein- oder mehrfach durch Niederalkyl, Hydroxyniederalkyl, Halogenniederalkyl, Niederalkenyl, 3- oder 4-gliedriges Niederalkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, $R_1$ Hydroxymethyl, mit einer gegebenenfalls substituierten Niederalkancarbonsäure verestertes oder mit einem Niederalkanol verethertes Hydroxymethyl, Formyl oder mit einem Niederalkanol acetalisiertes Formyl darstellt, $R_2$ Wasserstoff oder Niederalkyl bedeutet und $R_3$ einerseits N,N-Di-niederalkyl-amino, N-Cycloniederalkyl-N-niederalkyl-amino, N-Niederalkyl-N-phenylniederalkyl-amino, N,N-Di-cycloniederalkylniederalkyl-amino, N-Cycloniederalkyl-N-phenylniederalkyl-amino oder N,N-Di-phenylniederalkyl-amino bedeutet oder $R_3$ andererseits jeweils 5- bis 8-gliedriges Niederalkylenamino, Niederalkenylenamino, durch Monoaza, N'-Niederalkylmonoaza, Monooxa oder Monothia unterbrochenes Niederalkylenamino, durch Monoaza, N'-Niederalkylmonoaza, Monooxa oder Monothia unterbrochenes Niederalkenylenamino oder ein oder zwei ortho-anellierte Benzosysteme aufweisendes Niederalkylen- bzw. Niederalkenylen-amino darstellt, wobei Niederalkylen bzw. Niederalkenylen unverzweigt oder verzweigt sein und 4 bis 14, insbesondere 4 bis 7, C-Atome aufweisen kann und/oder ein oder zwei ortho-anellierte Benzosysteme aufweisen kann und Phenyl oder Benzo jeweils ein- oder mehrfach durch Niederalkyl, Hydroxyniederalkyl, Halogenniederalkyl, Niederalkenyl, 3- oder 4-gliedriges Niederalkylen, Niederalkoxy, Niederalkylthio, Niederalkansulfinyl, Niederalkansulfonyl, Hydroxy, Halogen, Niederalkanoyloxy, Niederalkanoyl und/oder Nitro substituiert oder unsubstituiert sein kann, und der aromatische Ring A ein- oder mehrfach durch Niederalkyl, Hydroxyniederalkyl, Halogenniederalkyl, Niederalkenyl, 3- oder 4-gliedriges Niederalkylen, Niederalkoxy, Niederalkylthio, Niederalkan-

BAD ORIGINAL

sulfinyl, Niederalkansulfonyl, Hydroxy, Halogen, Niederalkanoyloxy, Niederalkanoyl und/oder Nitro substituiert oder, bis auf $R_3$, unsubstituiert sein kann, oder ein Salz oder Isomeres davon herstellt.

8. Verfahren nach einem der Ansprüche 1-5, dadurch gekennzeichnet, dass man eine Verbindung der Formel

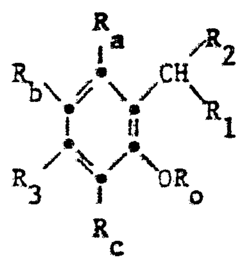

(Ia),

worin $R_o$ Wasserstoff oder Niederalkanoyl bedeutet,

$R_1$ Hydroxymethyl, Niederalkanoyloxymethyl, Niederalkoxymethyl, Formyl oder Diniederalkoxymethyl darstellt,

$R_2$ Wasserstoff oder Niederalkyl bedeutet,

$R_3$ einerseits N,N-Di-phenylniederalkyl-amino darstellt oder $R_3$ andererseits jeweils 5- bis 8-gliedriges Niederalkylenamino, durch Monooxa unterbrochenes Niederalkylenamino, oder ein ortho-anelliertes Benzosystem aufweisendes Niederalkylenamino bzw. Niederalkenylenamino bedeutet, und/oder $R_a$, $R_b$ und $R_c$ unabhängig voneinander Wasserstoff, Niederalkyl oder Halogen darstellen, oder ein Salz oder Isomeres davon herstellt.

9. Verfahren nach einem der Ansprüche 1-5, dadurch gekennzeichnet, dass man eine Verbindung der Formel Ia, worin $R_o$ Wasserstoff oder Niederalkanoyl bedeutet, $R_1$ Hydroxymethyl, Niederalkanoyloxymethyl, Niederalkoxymethyl, Formyl oder Diniederalkoxymethyl darstellt, $R_2$ Wasserstoff oder Niederalkyl darstellt, $R_3$ Di-niederalkyl-amino, 5- bis 8-gliedriges Niederalkylenamino, 5- bis 8-gliedriges Niederalkenylenamino oder 5- bis 8-gliedriges Monooxa-niederalkylenamino bedeutet und $R_a$ sowie $R_c$ Wasserstoff bedeuten und $R_b$ Halogen bedeutet,

BAD ORIGINAL

oder ein Salz oder Isomeres davon herstellt.

10. Verfahren nach einem der Ansprüche 1-5, dadurch gekennzeichnet, dass man eine Verbindung der Formel Ia, worin $R_o$ Wasserstoff oder Niederalkanoyl mit bis und mit 5 C-Atomen bedeutet, $R_1$ Hydroxymethyl, Niederalkanoyloxymethyl, Niederalkoxymethyl, Formyl oder Diniederalkoxymethyl darstellt, $R_2$ Niederalkyl mit bis und mit 4 C-Atomen bedeutet, $R_3$ 5- bis 7-gliedriges Niederalkylenamino, Morpholin-4-yl oder Pyrrol-1-yl darstellt, $R_a$ und $R_c$ jeweils Wasserstoff bedeuten und $R_b$ Niederalkyl mit bis und mit 4 C-Atomen oder Halogen bis und mit Atomnummer 35 darstellt, oder ein Salz oder Isomeres davon herstellt.

11. Verfahren nach einem der Ansprüche 1-5, dadurch gekennzeichnet, dass man eine Verbindung der Formel Ia, worin $R_o$ Niederalkanoyl mit bis und mit 5 C-Atomen darstellt, $R_1$ Hydroxymethyl, Niederalkoxymethyl mit bis und mit 5 C-Atomen oder Formyl bedeutet, $R_2$ Niederalkyl mit bis und mit 4 C-Atomen darstellt, $R_3$ Morpholin-4-yl oder Pyrrol-1-yl ist, $R_a$ und $R_c$ Wasserstoff bedeuten und $R_b$ Halogen bis und mit Atomnummer 35 oder Niederalkyl mit bis und mit 4 C-Atomen darstellt, oder ein Salz oder Isomeres davon herstellt.

12. Verfahren nach einem der Ansprüche 1-5, dadurch gekennzeichnet, dass man eine Verbindung der Formel Ia, worin $R_o$ Wasserstoff oder Niederalkanoyl mit bis und mit 5 C-Atomen darstellt, $R_1$ Hydroxymethyl, bedeutet, $R_2$ Niederalkyl mit bis und mit 4 C-Atomen darstellt, $R_3$ 5- bis 7-gliedriges Niederalkylenamino, Morpholin-4-yl oder Pyrrol-1-yl ist, $R_a$ und $R_c$ Wasserstoff bedeuten und $R_b$ Halogen bis und mit Atomnummer 35 oder Niederalkyl mit bis und mit 4 C-Atomen darstellt, oder ein Salz oder Isomeres davon herstellt.

13. Verfahren nach einem der Ansprüche 1-5, dadurch gekennzeichnet, dass man eine Verbindung der Formel Ia, worin $R_o$ Wasserstoff oder Niederalkanoyl mit bis und mit 5 C-Atomen darstellt, $R_1$ Formyl bedeutet, $R_2$ Niederalkyl mit bis und mit 4 C-Atomen darstellt, $R_3$ 5- bis 7-gliedriges Niederalkylenamino, Morpholin-4-yl oder Pyrrol-1-yl ist, $R_a$ und $R_c$ Wasserstoff bedeuten und $R_b$ Halogen bis und mit Atomnummer 35 oder Niederalkyl mit bis und mit 4 C-Atomen darstellt, oder ein Salz oder Isomeres davon herstellt.

14. Verfahren nach einem der Ansprüche 1-5, dadurch gekennzeichnet, dass man eine Verbindung der Formel Ia, worin $R_o$ Wasserstoff oder Niederalkanoyl mit bis und mit 5 C-Atomen bedeutet, $R_1$ Hydroxymethyl, Niederalkanoyloxymethyl, Niederalkoxymethyl, Formyl oder Diniederalkoxymethyl darstellt, $R_2$ Niederalkyl mit bis und mit 4 C-Atomen bedeutet, $R_3$ Pyrrol-1-yl darstellt, $R_a$ und $R_c$ jeweils Wasserstoff bedeuten und $R_b$ Niederalkyl mit bis und mit 4 C-Atomen oder Halogen bis und mit Atomnummer 35 darstellt, oder ein Salz oder Isomeres davon herstellt.

15. Verfahren nach einem der Ansprüche 1-5, dadurch gekennzeichnet, dass man eine Verbindung der Formel Ia, worin $R_o$, $R_a$ und $R_c$ jeweils Wasserstoff bedeuten, $R_b$ und $R_2$ jeweils Niederalkyl mit bis und mit 4 C-Atomen, wie Methyl, darstellen, $R_1$ Hydroxymethyl ist, und $R_3$ Pyrrol-1-yl bedeutet, herstellt.

16. Verfahren nach einem der Ansprüche 1-5, dadurch gekennzeichnet, dass man 2-(5-Chlor-2-hydroxy-4-morpholino-phenyl)-propan-1-ol herstellt.

17. Verfahren nach einem der Ansprüche 1-5, dadurch gekennzeichnet, dass man 2-[2-Hydroxy-5-methyl-4-(pyrrol-1-yl)-phenyl]-propan-1-ol herstellt.

18. Verfahren nach einem der Ansprüche 1-5, dadurch gekennzeichnet, dass man 2-[2-Hydroxy-5-methyl-4-(pyrrol-1-yl)-phenyl]-propanal oder ein Isomeres davon herstellt.

19. Verfahren nach einem der Ansprüche 1-5, dadurch gekennzeichnet, dass man 2-[5-Chlor-2-hydroxy-4-(piperidin-1-yl)-phenyl]-propanol,
2-[2-Acetoxy-5-chlor-4-(piperidin-1-yl)-phenyl]-propionaldehyd,
2-[2-Acetoxy-5-chlor-4-(piperidin-1-yl)-phenyl]-propanol,
2-[2-Acetoxy-5-chlor-4-(piperidin-1-yl)-phenyl]-propionaldehyd-diethylacetal,
2-[5-Chlor-2-hydroxy-4-(piperidin-1-yl)-phenyl]-propionaldehyd-diethylacetal,
2-[2-Acetoxy-5-chlor-4-(piperidin-1-yl)-phenyl]-1-methoxypropan,
2-[5-Chlor-2-hydroxy-4-(piperidin-1-yl)-phenyl]-1-methoxypropan,
1-Acetoxy-2-[2-acetoxy-5-chlor-4-(piperidin-1-yl)-phenyl]-propan,
1-Acetoxy-2-[5-chlor-2-hydroxy-4-(piperidin-1-yl)-phenyl]-ethan,
2-[5-Chlor-2-hydroxy-4-(piperidin-1-yl)-phenyl]-ethanol,
2-[2-Acetoxy-5-methyl-4-(pyrrol-1-yl)-phenyl]-ethanol,
2-[4-Dibenzylamino-2-hydroxy-5-methyl-phenyl]-propanol,
2-[4-Dibenzylamino-5-methyl-2-pivaloyloxy-phenyl]-1-pivaloyloxy-propan oder 2-[5-Methyl-2-pivaloyloxy-4-(pyrrol-1-yl)-phenyl]-1-pivaloyloxy-propan herstellt.

20. Verfahren zur Herstellung pharmazeutischer Präparate, dadurch gekennzeichnet, dass man eine Verbindung gemäss einem der Ansprüche 1, 6-19, gegebenenfalls unter Beimischung von üblichen Hilfs- und Trägerstoffen, zu pharmazeutischen Präparaten verarbeitet.

0107620

Europäisches Patentamt

# EUROPÄISCHER RECHERCHENBERICHT

Nummer der Anmeldung

EP 83 81 0462

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| Y | FR-A-2 007 554 (J.R. GEIGY)<br>* Ansprüche * | 1,18-20 | C 07 C 91/44<br>C 07 C 93/26<br>C 07 C 95/08<br>C 07 D 207/325<br>C 07 D 295/08<br>C 07 D 295/10<br>A 61 K 31/135<br>A 61 K 31/22<br>A 61 K 31/395 |
| Y | US-A-3 855 291 (S. ARCHER u.a.)<br>* Ansprüche; Spalte 1 * | 1,18-20 | |
| P,X | EP-A-0 082 109 (CIBA-GEIGY)<br>* Ansprüche; Seite 41; Beispiel 17 * | 1-18, 22-27 | |
| | | | **RECHERCHIERTE SACHGEBIETE (Int. Cl. 3)** |
| | | | C 07 C 91/00<br>C 07 C 93/00<br>C 07 C 95/00<br>C 07 D 207/00<br>C 07 D 295/00<br>A 61 K 31/00 |

Der vorliegende Recherchenbericht wurde für alle Patentansprüche erstellt.

| Recherchenort | Abschlußdatum der Recherche | Prüfer |
|---|---|---|
| DEN HAAG | 18-01-1984 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82